# EUROPÄISCHE PATENTSCHRIFT

(11) **EP 3 562 383 B1**
(45) Veröffentlichungstag und Bekanntmachung des Hinweises auf die Patenterteilung: **18.12.2024**
(21) Anmeldenummer: 17828902.1
(22) Anmeldetag: 21.12.2017
(51) Int. Cl.: A61B 5/024, A61B 5/16, A61B 5/026, A61B 5/349

(54) **VORRICHTUNG, SYSTEM UND VERFAHREN ZUR ERZEUGUNG VON BIOFEEDBACK**
DEVICE, SYSTEM AND METHOD FOR GENERATING BIOFEEDBACK
DISPOSITIF, SYSTÈME ET PROCÉDÉ POUR PRODUIRE UN BIOFEEDBACK

(30) Priorität: 29.12.2016 DE 102016015631
(43) Veröffentlichungstag der Anmeldung: 06.11.2019
(73) Patentinhaber: Mengden, Thomas, 44139 Dortmund (DE); AIT Austrian Institute of Technology GmbH, 1220 Wien (AT)
(72) Erfinder: MENGDEN, Thomas, 53859 Niederkassel/Rheidt (DE); WASSERTHEURER, Siegfried, 8344 Bad Gleichenberg (AT)
(74) Vertreter: Vossius & Partner Patentanwälte Rechtsanwälte mbB
(86) Internationale Anmeldenummer: PCT/EP2017/083976
(87) Internationale Veröffentlichungsnummer: WO 2018/122082

(56) Entgegenhaltungen:
- WO-A2-00/51677
- WO-A2-2006/100676
- DE-A1- 10 319 361
- US-A1- 2003 036 685
- US-A1- 2004 116 784
- US-B1- 6 554 763
- REGUIG F BEREKSI: "Photoplethysmogram signal analysis for detecting vital physiological parameters: An evaluating study", 2016 INTERNATIONAL SYMPOSIUM ON SIGNAL, IMAGE, VIDEO AND COMMUNICATIONS (ISIVC), IEEE, 21 November 2016 (2016-11-21), pages 167 - 173, XP033084496, DOI: 10.1109/ISIVC.2016.7893981

## Beschreibung

Die Erfindung betrifft Vorrichtungen, Systeme und Verfahren zur Erzeugung von Biofeedback. Die Erfindung betrifft insbesondere Vorrichtungen, Systeme und Verfahren, die Informationen über Veränderungen von Vitalparametern während einer Übung bereitstellen können, um die Wirksamkeit der Übung für einen Benutzer erkennbar zu machen.

Bluthochdruck stellt ein signifikantes Gesundheitsrisiko dar, das verschiedene Ursachen haben kann. Bei der Entstehung der essentiellen arteriellen Hypertonie können chronische Stressbelastungen eine wichtige Rolle spielen, da sie zu einer Verschiebung der vegetativen Balance zwischen Sympathikus und Parasympathikus führen können. Eine derartige Verschiebung der vegetativen Balance kann sich auf verschiedene Organe, beispielsweise das Herz-Kreislauf-System, auswirken und kann zu einer Erhöhung des Herzschlages, einer Steigerung der ventrikulären Kontraktion und einer Verengung der peripheren Gefäße führen. Ein derartiger Zustand kann zunächst akut und vorübergehend, später aber auch chronisch zu einer Blutdruckerhöhung führen, die das Risiko von Folgen wie Schlaganfall, Herzversagen und Nierenversagen erhöht.

Nicht-invasive Techniken zur Blutdrucksenkung sind attraktiv. Atemtechniken wie das Mantra-Atmen sind beispielhaft für Entspannungstechniken, mit denen jedenfalls bestimmte milde Formen von arterieller Hypertonie abgemildert werden können. Yoga oder andere Entspannungstechniken können ebenfalls zur Beeinflussung des Blutdrucks eingesetzt werden. Es ist wünschenswert, der Person, die eine Entspannungstechnik ausführt, eine Rückmeldung über deren Wirksamkeit zu geben. Dadurch kann die korrekte Ausführung überprüft und die Motivation gesteigert werden. Herkömmlich wird eine aus einem Elektrokardiogramm (EKG)-Signal abgeleitete Herzratenvariabilität (HRV) als Surrogatparameter herangezogen und ausgegeben, da diese leicht erfassbar ist. Bei derartigen herkömmlichen Systemen gibt es allerdings bislang keine Möglichkeit, die Akuteffekte auf den Blutdruck als Biofeedback auszugeben. Veränderungen der HRV müssen jedoch nicht notwendig mit Blutdruckveränderungen einhergehen. Letztere können beispielsweise bei einer Entspannungsübung erst mit deutlicher Verzögerung gegenüber einer Veränderung der Herz- oder Pulsrate auftreten.

Unter Verwendung einer Blutdruckmessmanschette kann der Blutdruck erfasst und als Biofeedback ausgegeben werden. Ein beispielhaftes System, bei dem eine Blutdruckmessmanschette eingesetzt wird, ist in der DE 10 2010 014 761 A1 beschrieben. Die Verwendung einer Blutdruckmessmanschette kann jedoch von Benutzern als störend empfunden werden und der Wirksamkeit der Entspannungsübung abträglich sein.

Veränderungen des Blutdruckes führen über eine veränderte transmurale Wandspannung der Arterien zu Veränderungen der Pulswellenlaufzeit ("Pulse Transit Time", PTT) und Pulswellengeschwindigkeit ("Pulse Wave Velocity", PWV) von Druck- oder Volumenpulsen im Blutkreislauf. Bei steigenden Blutdruckwerten nimmt die Pulswellengeschwindigkeit zu und die Pulswellenlaufzeit daher ab. Abfallenden Blutdruckwerten führen zu einer Zunahme der Pulswellenlaufzeit. Die Pulswellenlaufzeit kann als ein Parameter, der eine Blutdruckveränderung anzeigt, genutzt werden. Beispielhafte Systeme, die die Messung der Pulswellenlaufzeit erlauben, sind in der DE 10 2005 003 678 A1 und der DE 10 2010 014 761 A1 beschrieben. Das von derartigen Systemen bereitgestellte Biofeedback kann jedoch nur begrenzte Information über die Wirksamkeit einer Entspannungsübung liefern, da beispielsweise nicht erkennbar ist, durch welchen Wirkmechanismus im Körper eine erfasste Blutdruckveränderung herbeigeführt wird.

Die WO 2009/112000 A1 offenbart eine Vorrichtung zum Bestimmen der kardiovaskulären Variabilität, die nichtinvasiv aufgezeichnete Blutdrucksignale zur Analyse nutzt. Diese Vorrichtung dient zur klinischen Analyse aufgezeichneter Signale und ist nicht zur Bereitstellung von Biofeedback konfiguriert.

Die WO 2006/100676 A2 offenbart ein System und Verfahren zur kontinuierlichen Blutdrucküberwachung, das eine portable Überwachungseinrichtung mit einer Anzeige aufweist.

Die WO 00/51677 A2 offenbart ein Verfahren, bei dem Herzfrequenzvariabilitäts (HRV)-Informationen empfangen werden, Leistung in der HRV über einen ersten Frequenzbereich bestimmt wird und eine Leistungsspitze in dem ersten Frequenzbereich ausgewählt wird.

Der vorliegenden Erfindung liegt die Aufgabe zugrunde, Vorrichtungen, Systeme und Verfahren bereitzustellen, die Verbesserungen im Hinblick auf wenigstens einige der genannten Nachteile herkömmlicher Systeme bieten. Insbesondere liegt der vorliegenden Erfindung die Aufgabe zugrunde, Vorrichtungen, Systeme und Verfahren bereitzustellen, die bei Durchführung einer Entspannungsübung zusätzliche Informationen ableiten können, die für die Wirksamkeit der Entspannungsübung von Relevanz sein können.

Erfindungsgemäß werden eine Vorrichtung, ein System und ein Verfahren mit den in den unabhängigen Patentansprüchen definierten Merkmalen angegeben. Die abhängigen Patentansprüche definieren Ausführungsformen.

Eine erfindungsgemäße Vorrichtung zur Erzeugung von Biofeedback weist wenigstens eine Schnittstelle zum Empfangen eines Pulswellensignals, das einen Druck- oder Volumenpuls einer Pulswelle in einem Blutkreislauf als Funktion der Zeit repräsentiert, und eines EKG-Signals auf. Die Vorrichtung weist eine Auswerteeinrichtung auf, die eingerichtet ist, basierend auf dem Pulswellensignal und dem EKG-Signal eine Pulswellenlaufzeit zu bestimmen und/oder eine Auswertung einer Pulswellenform des Pulswellensignals auszuführen und das Biofeedback abhängig von der Pulswellenlaufzeit und/oder der Auswertung der Pulswellenform zu erzeugen. Die Vorrichtung weist eine Ausgabeschnittstelle zum Bereitstellen des Biofeedbacks auf.

Die Vorrichtung kann derart ausgestaltet sein, dass sie im Betrieb nicht nur eine Pulswellenlaufzeit ermittelt, aus der sich Blutdruckveränderungen erkennen lassen können, sondern auch eine Auswertung der Pulswellenform des erfassten Druck- oder Volumenpulses vornehmen kann. Aussagekräftigeres Biofeedback kann dadurch an den Benutzer bereitgestellt werden. Durch die Analyse der Pulswellenform lassen sich typische hämodynamische Veränderungen erkennen. Anhand von Veränderungen der Peaks der Pulswellenform kann die Vorrichtung beispielsweise automatisch Aussagen darüber treffen, ob sich nur die kardiale Pumpleistung und Herzfrequenz verändert haben, oder ob auch der periphere Widerstand von Gefäßen erniedrigt wurde.

Die Pulswellenlaufzeit ist invers proportional zur Pulswellengeschwindigkeit. Entsprechend werden durch die Erfassung der Pulswellenlaufzeit auch Informationen über die Pulswellengeschwindigkeit ermittelt. Die Erzeugung von Biofeedback abhängig von der Pulswellenlaufzeit kann auf verschiedene Weise erfolgen, wobei insbesondere auch die Bestimmung einer Pulswellengeschwindigkeit aus der Pulswellenlaufzeit und eine weitere Auswertung der Pulswellengeschwindigkeit mit umfasst ist.

Aufgrund des Zusammenhangs zwischen der Pulswellenlaufzeit und der Pulswellengeschwindigkeit wird nachfolgend nur noch auf die Pulswellenlaufzeit Bezug genommen, wobei es sich versteht, dass eine Abhängigkeit von Biofeedback von der Pulswellenlaufzeit im Sinne dieser Anmeldung auch eine Abhängigkeit des Biofeedbacks von der Pulswellengeschwindigkeit impliziert, und dass eine Abhängigkeit einer physiologischen Größe von der Pulswellenlaufzeit im Sinne dieser Anmeldung auch eine Abhängigkeit der physiologischen Größe von der Pulswellengeschwindigkeit impliziert.

Die Auswerteeinrichtung kann eingerichtet sein, durch die Auswertung der Pulswellenform einen einer Blutdruckveränderung zugrundeliegenden Wirkmechanismus, eine Magnitude der Blutdruckveränderung, eine Veränderung einer kardialen Pumpleistung und/oder eine Veränderung einer Pulswellenreflektion zu bestimmen. Dazu kann die Auswerteeinrichtung beispielsweise anhand von mehreren erfassten Druck- oder Volumenpulsen ermitteln, wie sich die Lage und/oder Höhe von Peaks des Pulswellensignals mit zunehmender Dauer der Entspannungsübung verändert. Typische hämodynamische Veränderungen lassen sich auf diese Weise erkennen, ohne dass beispielsweise die Verwendung einer Blutdruckmessmanschette zwingend erforderlich ist.

Die Auswerteeinrichtung ist eingerichtet, durch die Auswertung der Pulswellenform zu ermitteln, ob eine durch eine Vagussteigerung oder eine Sympathikusdämpfung verursachte Blutdruckveränderung vorliegt. Beispielsweise kann es wünschenswert sein, eine Blutdruckveränderung spezifisch durch Aktivierung des Parasympathikus (Vagusaktivierung) zur Verbesserung der vegetativen Balance oder durch Sympathikusdämpfung zur Verringerung des peripheren Widerstands herbeizuführen. Entsprechend kann die Vorrichtung den der Blutdruckveränderung zugrundeliegenden Wirkmechanismus erkennen, beispielsweise durch automatische Unterscheidung von Vagusaktivierung und Sympathikusdämpfung, und kann ein entsprechendes Biofeedback bereitstellen.

Die Auswerteeinrichtung kann eingerichtet sein, über die Ausgabeschnittstelle anzuzeigen, ob eine durch eine Vagussteigerung oder eine Sympathikusdämpfung verursachte Blutdruckveränderung vorliegt. Auf diese Weise kann einem Benutzer, der beispielsweise bestrebt ist, zur Verringerung des peripheren Widerstands eine Blutdruckverringerung durch Sympathikusdämpfung zu bewirken, aussagekräftiges Biofeedback zur Verfügung gestellt werden.

Die Auswerteeinrichtung kann eingerichtet sein, eine Veränderung wenigstens einer der folgenden Größen zu erkennen, um zu ermitteln, ob eine durch eine Vagussteigerung oder eine Sympathikusdämpfung verursachte Blutdruckveränderung vorliegt: Dauer einer frühen systolischen Phase; Druck in der frühen systolischen Phase; und/oder Dauer einer späten systolischen Phase. Veränderungen dieser Größen lassen sich einfach, beispielsweise von Herzschlag zu Herzschlag, erkennen. Aus einem Vergleich dieser Größen für mehrere kardiale Zyklen ist ermittelbar, ob der periphere Widerstand der Gefäße verringert wird, wenn die späte systolische Phase verlängert wird. Aus einem Vergleich dieser Größen für mehrere kardiale Zyklen ist auch eine Veränderung der kardialen Pumpleistung erkennbar.

Die Auswerteeinrichtung kann eingerichtet sein, die Bestimmung der Pulswellenlaufzeit und/oder die Auswertung der Pulswellenform jeweils für jeden Herzzyklus einer Mehrzahl aufeinanderfolgender Herzzyklen auszuführen. Dadurch kann zeitaufgelöst pro Herzschlag erkannt werden, wie sich die Laufzeit des Druck- oder Volumenpulses vom Herzen bis zu seiner Erfassung durch einen Sensor sowie die Laufzeit des von den peripheren Gefäßen reflektierten Druck- oder Volumenpulses verändert.

Die Vorrichtung kann eingerichtet sein, für jeden Herzzyklus der Mehrzahl aufeinanderfolgender Herzzyklen jeweils das Biofeedback über die Ausgabeschnittstelle auszugeben. Dadurch kann dem Benutzer zeitaufgelöstes Biofeedback bereitgestellt werden.

Das Biofeedback kann wenigstens eine Information beinhalten, die ausgewählt ist aus der Gruppe bestehend aus: einer Blutdruckveränderung, einem Blutdruck, einer kardialen Pumpleistung, einer Pulswellenreflektion und einem der Blutdruckveränderung zugrundeliegenden Wirkmechanismus. Diese Information erlaubt dem Benutzer, die Wirksamkeit der Entspannungsübung zur Blutdrucksenkung, beispielsweise durch Vagusaktivierung oder Sympathikusdämpfung, zu überprüfen.

Die Vorrichtung kann eingerichtet sein, über die Ausgabeschnittstelle sowohl das Biofeedback als auch Anweisungen zur Durchführung der Entspannungsübung, insbesondere Atemanweisungen, auszugeben. Dadurch kann mit nur einem Gerät, das beispielsweise als Handgerät ausgestaltet sein kann, der Benutzer zur Durchführung der Entspannungsübung angeleitet werden und kann anhand des Biofeedbacks eine Beurteilung der Wirksamkeit vornehmen.

Die Vorrichtung kann eingerichtet sein, die Anweisungen zur Durchführung der Entspannungsübung abhängig von der Pulswellenlaufzeit und/oder der Auswertung der Pulswellenform zu erzeugen. Dadurch kann in Art einer Rückkopplungsschleife das Verhalten des Benutzers während der Entspannungsübung gezielt beeinflusst werden.

Die Ausgabeschnittstelle kann eine optische Ausgabeeinheit aufweisen. Die Vorrichtung kann eingerichtet sein, die Anweisungen zur Durchführung der Entspannungsübung und das Biofeedback gleichzeitig über die optische Ausgabeeinheit auszugeben. Die optische Ausgabeeinheit kann beispielsweise ein Bildschirm eines Handgeräts sein. Dadurch kann dem Benutzer graphisch ein Eindruck von der Wirksamkeit der Entspannungsübung vermittelt werden. Die Ausgabeschnittstelle kann alternativ oder zusätzlich eine akustische Ausgabeeinheit, beispielsweise einen Lautsprecher, aufweisen.

Die Auswerteeinrichtung kann eingerichtet sein, die Pulswellenlaufzeit und/oder wenigstens eine durch die Auswertung der Pulswellenform ermittelte Kenngröße der Pulswellenform als Funktion eines Parameters der Entspannungsübung, insbesondere einer Atemfrequenz, zu bestimmen. Dadurch kann die Ausführung der Entspannungsübung, beispielsweise durch allmähliche Absenkung der Atemfrequenz beim Mantra-Atmen, mit der resultierenden Veränderung von Vitalparametern korreliert werden.

Die Vorrichtung kann eine mobile, insbesondere eine tragbare Vorrichtung sein. Die Vorrichtung kann als Handgerät ausgebildet sein. Die Vorrichtung kann eine tragbare Kommunikationsvorrichtung mit einer Kommunikationsschnittstelle zur Kommunikation mit einem Weiterverkehrsnetz oder einem zellulären Kommunikationsnetz sein. Die Vorrichtung kann ein Mobiltelefon, ein Tablet-Computer oder ein tragbarer Computer sein, um Signale von Sensoren zu empfangen und sowohl die Pulswellenlaufzeit zu ermitteln als auch eine Auswertung der Pulswellenform vorzunehmen.

Ein erfindungsgemäßes System weist die Vorrichtung zur Erzeugung von Biofeedback nach einem Ausführungsbeispiel auf. Das System weist eine Sensoreinrichtung auf, die mit der wenigstens einen Schnittstelle der Vorrichtung koppelbar ist und zum Erfassen des Pulswellensignals eingerichtet ist.

Die Sensoreinrichtung kann einen ersten Sensor zum Erfassen des Pulswellensignals und einen zweiten Sensor zum Erfassen des EKG-Signals aufweisen. Der erste Sensor kann eingerichtet sein, um durch Infrarotlichttransmission oder Infrarotlichtreflexion das Pulswellensignal zu erfassen. Der erste Sensor kann eine Photoplethysmographieeinrichtung umfassen. Der erste Sensor kann eingerichtet sein, um beispielsweise an einem Finger, einem Handgelenk oder einem Arm reversibel lösbar angebracht zu werden.

Der erste Sensor kann eingerichtet sein, um das Pulswellensignal in Datenpaketen oder Datenrahmen an die Vorrichtung zu übertragen. Der erste Sensor kann eine Schnittstelle zur drahtgebundenen oder drahtlosen paketierten Datenübertragung aufweisen, um das Pulswellensignal an die Vorrichtung zu übertragen. Ähnlich kann der zweite Sensor eingerichtet sein, um das EKG-Signal in Datenpaketen oder Datenrahmen an die Vorrichtung zu übertragen. Der zweite Sensor kann eine Schnittstelle zur drahtgebundenen oder drahtlosen paketierten Datenübertragung aufweisen, um das EKG-Signal an die Vorrichtung zu übertragen.

Das System kann eine Steuereinheit zum Steuern des ersten Sensors und des zweitens Sensors und zum Synchronisieren des von dem ersten Sensor erfassten Pulswellensignals und des von dem zweiten Sensor erfassten EKG-Signals aufweisen.

Ein Verfahren zur Erzeugung von Biofeedback, insbesondere bei einer Entspannungsübung zur Blutdrucksenkung, weist die folgenden Schritte auf: Empfangen eines Pulswellensignals, das einen Druck- oder Volumenpuls einer Pulswelle in einem Blutkreislauf als Funktion der Zeit repräsentiert, und eines EKG-Signals an einer Schnittstelle; Bestimmen einer Pulswellenlaufzeit durch Auswertung des Pulswellensignals und des EKG-Signals und/oder Auswerten einer Pulswellenform des Pulswellensignals; Erzeugen des Biofeedbacks abhängig von der Pulswellenlaufzeit und/oder der Auswertung der Pulswellenform; und Ausgeben des Biofeedbacks.

Mit dem Verfahren wird nicht nur eine Pulswellenlaufzeit ermittelt, aus der sich Blutdruckveränderungen erkennen lassen können, sondern es erfolgt auch eine Auswertung der Pulswellenform des erfassten Druck- oder Volumenpulses. Aussagekräftigeres Biofeedback kann dadurch an den Benutzer bereitgestellt werden. Durch die Analyse der Pulswellenform lassen sich typische hämodynamische Veränderungen erkennen. Anhand von Veränderungen der Peaks der Pulswellenform kann die Vorrichtung beispielsweise automatisch Aussagen darüber treffen, ob sich nur die kardiale Pumpleistung und Herzfrequenz verändert haben, oder ob auch der periphere Widerstand von Gefäßen erniedrigt wurde.

Das Biofeedback kann während einer geführten Entspannungsübung zur Blutdrucksenkung, insbesondere während einer Atemübung, erzeugt werden.

Durch das Auswerten der Pulswellenform wird ermittelt, ob eine Blutdruckveränderung durch eine Vagussteigerung oder eine Sympathikusdämpfung verursacht wird. Das ausgegebene Biofeedback kann anzeigen, ob die Blutdruckveränderung durch eine Vagussteigerung oder eine Sympathikusdämpfung verursacht wird.

Das Verfahren kann von der Vorrichtung oder dem System nach einem Ausführungsbeispiel ausgeführt werden.

Weitere optionale Merkmale des Verfahrens und die dadurch jeweils erzielten Wirkungen entsprechen den unter Bezugnahme auf Ausführungsbeispiele der Vorrichtung beschriebenen Merkmalen und Wirkungen.

Bevorzugte Ausführungsformen der Erfindung werden nachfolgend mit Bezug auf die Figuren im Detail beschrieben. Es zeigen:
- Fig. 1: eine schematische Darstellung eines Systems nach einem Ausführungsbeispiel;
- Fig. 2: eine schematische Darstellung eines Pulswellensignals und eines EKG-Signals zur Erläuterung der Funktionsweise der Vorrichtung nach einem Ausführungsbeispiel;
- Fig. 3: eine schematische Darstellung einer Pulswellenform eines Pulswellensignals zur Erläuterung der Funktionsweise der Vorrichtung nach einem Ausführungsbeispiel;
- Fig. 4: eine schematische Darstellung einer Pulswellenform eines Pulswellensignals zur Erläuterung der Funktionsweise der Vorrichtung nach einem Ausführungsbeispiel;
- Fig. 5: eine schematische Darstellung einer Pulswellenform eines Pulswellensignals zur Erläuterung der Funktionsweise der Vorrichtung nach einem Ausführungsbeispiel;
- Fig. 6: eine schematische Darstellung von Signalformen zur Erläuterung der Funktionsweise der Vorrichtung nach einem Ausführungsbeispiel;
- Fig. 7: ein Flussdiagramm eines Verfahrens nach einem Ausführungsbeispiel;
- Fig. 8: eine schematische Darstellung einer optischen Ausgabeeinheit zur Bereitstellung von Biofeedback bei einer Vorrichtung nach einem Ausführungsbeispiel;
- Fig. 9: eine Veränderung einer Herzrate und einer Pulswellenlaufzeit während einer Entspannungsübung;
- Fig. 10: ein Blockdiagramm eines Systems nach einem Ausführungsbeispiel;
- Fig. 11: eine schematische Darstellung einer optischen Ausgabeeinheit zur Bereitstellung von Biofeedback bei einer Vorrichtung nach einem Ausführungsbeispiel;
- Fig. 12: eine weitere schematische Darstellung der optischen Ausgabeeinheit von Fig. 11;
- Fig. 13: eine weitere schematische Darstellung der optischen Ausgabeeinheit von Fig. 11;
- Fig. 14: eine Darstellung zur Erläuterung von Techniken zum Erzeugen von Biofeedback; und
- Fig. 15: ein Flussdiagramm eines Verfahrens nach einem Ausführungsbeispiel.

Nachfolgend werden bevorzugte und vorteilhafte Ausführungsbeispiele der Erfindung unter Bezugnahme auf die Figuren beschrieben, in denen identische Bezugszeichen identische oder ähnliche Elemente bezeichnen. Die Merkmale der verschiedenen Ausführungsbeispiele können miteinander kombiniert werden, sofern dies in der nachfolgenden Beschreibung nicht ausdrücklich ausgeschlossen wird.

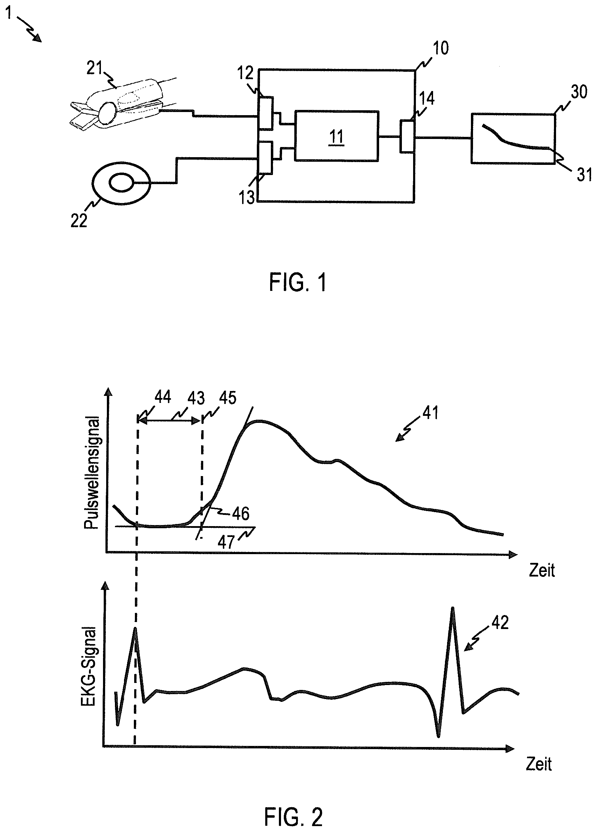

Fig. 1 zeigt eine schematische Darstellung eines Systems 1 nach einem Ausführungsbeispiel. Das System 1 umfasst einen Sensor oder mehrere Sensoren 21, 22 zum Erfassen eines Pulswellensignals und eines EKG-Signals. Das System 1 umfasst eine Vorrichtung 10 zur Erzeugung von Biofeedback. Die Vorrichtung 10 kann derart eingerichtet sein, dass sie aus dem Pulswellensignal und dem EKG-Signal eine Pulswellenlaufzeit eines Druck- oder Volumenpulses im Blutkreislauf ermittelt und zusätzlich eine Auswertung einer Pulswellenform des Pulswellensignals vornimmt, um Biofeedback zu erzeugen. Die Vorrichtung 10 kann das Biofeedback über eine in die Vorrichtung 10 baulich integrierte oder davon separate optische Ausgabeeinheit 30 ausgeben.

Die Sensoren 21, 22 können für eine zerstörungsfrei reversibel lösbare Befestigung an einem menschlichen Körper eingerichtet sein. Die Sensoren 21, 22 können verschiedene Messtechniken einsetzen, um das Pulswellensignal und das EKG-Signal zu erfassen. Derartige Messtechniken sind dem Fachmann an sich bekannt und werden deswegen hier nicht ausführlich beschrieben. Beispielsweise kann der Sensor 21 zum Erfassen eines Druck- oder Volumenpulses einer Pulswelle in einem Blutkreislauf eine Photoplethysmographieeinrichtung umfassen. Der Sensor 21 zum Erfassen des Druck- oder Volumenpulses kann zur lösbaren Befestigung an einem Finger ausgebildet sein. Der Sensor 21 zum Erfassen des Druck- oder Volumenpulses kann als eine an einem Handgelenk befestigbare Einheit ausgebildet oder zusammen mit anderen Sensoren in eine derartige Einheit integriert sein. Der Sensor 21 zum Erfassen des Druck- oder Volumenpulses kann auch in eine Einheit integriert sein, die weitere Funktionen aufweist, beispielsweise in einen Kopfhörer, der in oder an einem Ohr befestigbar ist. Der Sensor 22 zum Erfassen des EKG-Signals kann in einem Brustgurt oder einer anderen Halteeinrichtung integriert sein oder kann eine adhäsive Fläche zur Befestigung aufweisen. Die Sensoren 21, 22 können über eine drahtgebundene oder drahtlose Verbindung mit einer Vorrichtung 10 zur Erzeugung von Biofeedback gekoppelt sein.

Die Vorrichtung 10 weist wenigstens eine Schnittstelle 12, 13 auf, um das EKG-Signal und das Pulswellensignal von den Sensoren 21, 22 zu empfangen. Die wenigstens eine Schnittstelle 12, 13 kann für eine drahtgebundene oder drahtlose Kommunikation mit den Sensoren 21, 22 eingerichtet sein. Die wenigstens eine Schnittstelle 12, 13 kann eine USB- oder Bluetooth-Schnittstellen sein. Die wenigstens eine Schnittstelle 12, 13 kann eingerichtet sein, das EKG-Signal und das Pulswellensignal in Datenpaketen oder Datenrahmen zu empfangen, und um die Datenübertragung und Datenerfassung zwischen den Sensoren 21, 22 zu synchronisieren.

Die Vorrichtung 10 weist eine Auswerteeinrichtung 11 zum Auswerten des EKG-Signals und des Pulswellensignals auf. Die Auswerteeinrichtung 11 kann wenigstens eine integrierte Halbleiterschaltung aufweisen. Die Auswerteeinrichtung 11 kann eine anwendungsspezifische Spezialschaltung, einen Controller, einen Mikrocontroller, einen Prozessor, einen Mikroprozessor oder eine beliebige Kombination dieser oder anderer integrierter Halbleiterschaltungen aufweisen. Die Auswerteeinrichtung 11 ist eingerichtet, das EKG-Signal und das Pulswellensignal zur Erzeugung von Biofeedback auszuwerten. Die Auswerteeinrichtung 11 ist eingerichtet, aus dem EKG-Signal und dem Pulswellensignal eine Pulslaufzeit des Druck- oder Volumenpulses vom Herzen bis zum Sensor 21 zu ermitteln. Die Auswerteeinrichtung 11 ist eingerichtet, durch eine Auswertung einer Pulswellenform des Pulswellensignals zusätzlich zur Pulswellenlaufzeit weitere Informationen automatisch zu ermitteln. Diese weiteren Informationen können beispielsweise eine Veränderung einer kardialen Pumpleistung während einer Entspannungsübung beinhalten, die aus einer Veränderung einer Amplitude von Druck- oder Volumenpulsen während der Entspannungsübung ermittelbar ist. Alternativ oder zusätzlich kann eine Veränderung eines peripheren Widerstands des Blutkreislaufs während der Entspannungsübung erkannt werden, die jeweils aus einer relativen Lage zweier Peaks eines Druck- oder Volumenpulsen ermittelbar ist. Alternativ oder zusätzlich kann erkannt werden, ob eine Blutdruckveränderung über den Sympathikus oder den Parasympathikus (z.B. Vagus) verursacht wird. Beispiele für andere Informationen, die aus der Analyse der Pulswellenform ermittelbar sind, beinhalten die Veränderung des Widerstands peripherer Blutgefäße oder die Veränderung der Pulswellenreflektion im Blutkreislauf.

Die Auswerteeinrichtung 11 ist eingerichtet, Biofeedback zu erzeugen und über eine Ausgabeschnittstelle auszugeben. Das Biofeedback kann beispielsweise über eine Schnittstelle 14 zu einer optischen Anzeigeeinheit 30 übertragen und von dieser ausgegeben werden. Das Biofeedback kann die Veränderung eines oder mehrerer Vitalparameter während einer Entspannungsübung als Funktion der Zeit und/oder als Funktion eines die Durchführung der Entspannungsübung charakterisierenden Parameters, beispielsweise einer Atemfrequenz, angeben. Das Biofeedback kann Information über eine Blutdruckveränderung, eine kardiale Pumpleistung, eine Pulswellenreflektion und/oder einen der Blutdruckveränderung zugrundeliegenden Wirkmechanismus beinhalten, ohne darauf beschränkt zu sein.

Die Vorrichtung 10 kann eingesetzt werden, um Biofeedback während der Durchführung einer Entspannungsübung auszugeben. Dabei kann die Vorrichtung 10 nicht nur das Biofeedback zur Ausgabe über eine Ausgabeschnittstelle erzeugen, sondern auch Anweisungen zur Durchführung der Entspannungsübung. Atemanweisungen sind beispielhaft für derartige Anweisungen. Die Anweisungen zur Durchführung der Entspannungsübung können abhängig von der zeitabhängigen Veränderung der Pulswellenlaufzeit und/oder abhängig von der Auswertung der Pulswellenform erzeugt werden. Beispielsweise können abhängig von der Auswertung der Pulswellenform die Anweisungen derart erzeugt werden, dass eine Blutdruckveränderung durch Sympathikusdämpfung oder durch Vagussteigerung wahrscheinlicher wird.

Eine nicht beschränkende Anwendung der Vorrichtung 10 wird nachfolgend im Kontext von Mantra-Atmen beschrieben, wobei die Vorrichtung 10 nicht auf diese Anwendung beschränkt ist. Das tiefe und langsame Mantra-Atmen stellt eine Methode zur Blutdrucksenkung dar. Mantra-Atmen mit Atemfrequenzen von sechs Atemzügen pro Minute führt akut und chronisch zu einer Sensibilisierung des Baroreflexes und damit zu einer Blutdrucksenkung. Darüber hinaus kommt es zu einer Aktivierung des Parasympathikus (Vagus) und damit zu einer Verbesserung der vegetativen Balance. Hierdurch können der Blutdruck und die Herzfrequenz gesenkt werden. Als direkt, nicht-invasiv und kontinuierlich messbarer Parameter kann die Pulswellenlaufzeit von der Vorrichtung 10 ermittelt und als akutes Feedback zur Blutdruckveränderung ausgegeben werden. Die Änderung der Pulswellenlaufzeit kann abhängig vom Benutzer unterschiedlich sein, so dass die Veränderung der Pulswellenlaufzeit isoliert betrachtet von mehreren kurzfristig wirkenden Faktoren der Kreislaufsteuerung beeinflusst werden kann. Durch eine Analyse der Pulswellenform des Pulswellensignals, das einen Druck- oder Volumenpuls im Blutkreislauf repräsentiert, kann die Vorrichtung 10 zusätzliches und noch aussagekräftigeres Biofeedback erzeugen. Die Aufzeichnung und Analyse der Pulswellenform sowie der Pulswellenlaufzeit vom Herzen in die Peripherie, beispielsweise zu einem Finger, erlaubt es, die Effekte von verschiedenen Entspannungstechniken auf den Blutdruck zu erfassen. Die Analyse der Pulswellenform ermöglicht eine Aussage über den Wirkmechanismus sowie Rückschlüsse auf die Magnitude der Blutdruckveränderung. Die Aufzeichnung und Analyse der Pulswellenform sowie der Pulswellenlaufzeit kann während der Entspannungsübung kontinuierlich oder quasi-kontinuierlich erfolgen, so dass das Biofeedback bei jedem Herzschlag aktualisiert werden kann.

Die von der Vorrichtung 10 durchgeführte Ermittlung der Pulswellenlaufzeit und Analyse der Pulswellenform wird anhand beispielhafter Signalformen unter Bezugnahme auf Fig. 2 bis Fig. 5 näher beschrieben.

Fig. 2 zeigt eine schematische Darstellung eines Pulswellensignals 41 und eines EKG-Signals 42. Die Vorrichtung 10 kann das Pulswellensignal 41 und das EKG-Signal 42 von dem wenigstens einen Sensor 21, 22 empfangen, beispielsweise in Form von Datenpaketen oder Datenrahmen. Zur Ermittlung der Pulswellenlaufzeit kann die Vorrichtung 10 einen Zeitpunkt 44 einer R-Zacke des EKG-Signals 42 ermitteln. Die R-Zacke des EKG-Signals 42 zeigt den Beginn der Ausbreitung der Druck- oder Volumenpulses im Blutkreislauf vom Herzen aus an. Um die Ankunftszeit des Druck- oder Volumenpulses in einem Teil des Blutkreislaufs, an dem der Sensor 21 den Druck- oder Volumenpuls erfasst, zu ermitteln, kann die Vorrichtung 10 einen Zeitpunkt bestimmen, an dem die ansteigende Flanke des Pulswellensignal 41 beginnt. Dies kann beispielsweise durch einen Schwellenwertvergleich erfolgen. Bei einer Ausgestaltung kann zur genaueren Ermittlung der Pulswellenlaufzeit ein Schnittpunkt zwischen einer Tangente 46 an die ansteigende Flanke des Pulswellensignals 41 und einer durch das Minimum der J-Kurve des Pulswellensignals 41 verlaufenden Geraden 47 ermittelt werden. Der Zeitpunkt 45, dem dieser Schnittpunkt entspricht, repräsentiert die Ankunftszeit des von dem Sensor 21 erfassten Druck- oder Volumenpulses. Die Pulswellenlaufzeit 43 kann als Zeitdifferenz zwischen der R-Zacke des EKG-Signals 42 und der Ankunftszeit des von dem Sensor 21 erfassten Druck- oder Volumenpulses ermittelt werden.

Die Vorrichtung 10 kann die Pulswellenlaufzeit 43 für jeden von mehreren Druck- oder Volumenpulsen bestimmen, der durch eine Mehrzahl sequentieller Herzschläge ausgelöst wird. Die Vorrichtung 10 kann die Pulswellenlaufzeit 43 während der Durchführung einer Entspannungsübung individuell für jeden Herzschlag ermitteln. Als Biofeedback kann von Herzschlag zu Herzschlag jeweils die ermittelte Pulswellenlaufzeit oder die dazu korrespondierende Änderung des Blutdrucks, beispielsweise relativ zum Beginn der Entspannungsübung, ausgegeben werden. Optional kann eine weitere Verarbeitung, beispielsweise durch Berechnung eines gleitenden Mittelwerts oder durch Ausfiltern messtechnischer Ausreißwerte, erfolgen, um das Biofeedback zu erzeugen.

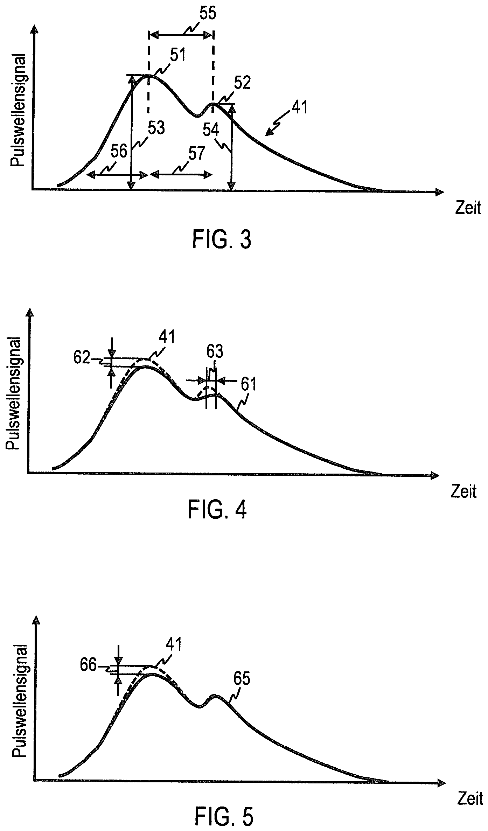

Fig. 3, Fig. 4 und Fig. 5 zeigen jeweils schematische Darstellung einer Pulswellenform eines Pulswellensignals 41, 61, 65. Die Vorrichtung 10 kann das Pulswellensignal 41, 61, 65 von dem Sensor 21 empfangen, beispielsweise in Form von Datenpaketen oder Datenrahmen. Das Pulswellensignal 41, 61, 65 repräsentiert den Druck- oder Volumenpuls, der von dem Sensor 21 als Funktion der Zeit erfasst wurde. Dargestellt ist jeweils nur die Pulsform des Pulswellensignals, das genau einem durch einen einzigen Herzschlag verursachten Druck- oder Volumenpuls entspricht. Die Pulswellenform des Pulswellensignals 41, 61, 65 weist typischerweise einen ersten Peak 51 und einen zweiten Peak 52 auf. Der zweite Peak 52 wird durch die Reflexion des vom Herzen ausgehenden Druck- oder Volumenpulses in peripheren Blutgefäßen verursacht. Die Pulswellenform hängt beispielsweise von der kardialen Pumpleistung und dem Widerstand peripherer Gefäße ab. So beeinflusst die kardiale Pumpleistung beispielsweise eine Amplitude 53 des ersten Peaks 51. Der Widerstand peripherer Gefäße beeinflusst eine Amplitude 54 des zweiten Peaks 52 sowie einen Zeitabstand 55 zwischen den Peaks 51, 52. Durch Veränderung des Widerstands von Gefäßen kann beispielsweise die Dauer einer frühen Systole 56 und/oder die Dauer der späten Systole 57 beeinflusst werden.

Die Veränderung der Pulswellenform bei einer Entspannungsübung ermöglicht es, Rückschlüsse zu ziehen, die zur Verwendung als Biofeedback geeignet sind. So führt beispielsweise eine über den Sympathikus ausgelöste Blutdruckerhöhung zu folgenden Veränderungen: Erhöhung des peripheren Widerstandes und Erhöhung der Nachlast; Erhöhung der kardialen Pumpleistung; sowie Erhöhung der Herzfrequenz. Diese hämodynamischen Veränderungen spiegeln sich dementsprechend auch in der Pulswellenform und führen zu folgenden Veränderungen: Verkürzung der frühen systolischen Phase 56; Erhöhung des Drucks in der frühen systolischen Phase 56; sowie frühere Pulswellenreflektion und Verkürzung der "späten" Systole 57, welche mit einer Veränderung der Pulswellenkontur in diesem Bereich einhergeht. Im Unterschied dazu führt eine über den Vagus ausgelöste Blutdruckerhöhung (Vagusdämpfung) zu folgenden Veränderungen: Erhöhung der Herzfrequenz sowie Erhöhung der kardialen Pumpleistung.

Die durch den Vagus ausgelösten hämodynamischen Veränderungen verändern die Pulswellenform somit anders als die durch den Sympathikus ausgelösten hämodynamischen Veränderungen, da der periphere Widerstand und damit die Nachlast durch den Vagus nicht beeinflusst wird. Die späte Systole wird bei einer Vagussteigerung oder Vagusdämpfung nur unwesentlich beeinflusst.

Eine durch eine Entspannungstechnik wie Mantra-Atmen herbeigeführte Blutdrucksenkung kann durch einen Anstieg der Pulswellenlaufzeit unspezifisch erfasst werden. Darüber hinaus kann der zugrunde liegende Mechanismus durch eine zusätzliche Analyse der Pulswellenkontur erfasst werden. Insbesondere kann auf diese Weise unterschieden werden, ob eine Blutdruckveränderung durch eine Vagussteigerung oder eine Sympathikusdämpfung verursacht wird. Der Benutzer kann über eine Schnittstelle Information abrufen, die angibt, ob über mehrere Entspannungsübungen hinweg eine Senkung des Widerstands peripherer Gefäße erreicht wurde. Eine derartige Information muss nicht während der Entspannungsübung ausgegeben werden, sondern kann beispielsweise als Bestandteil eines erst nach mehreren Übungen erstellten Berichts für den Probanden oder eine den Probanden betreuenden Fachperson erzeugt und ausgegeben werden.

Fig. 4 zeigt eine Pulswellenform 61 eines Pulswellensignals, das während einer Entspannungsübung erfasst wird, bei der eine Sympathikusdämpfung erreicht wird. Die Pulswellenform des zu Beginn der Entspannungsübung erfassten Pulswellensignals 41, das als Referenz für die Analyse der Veränderungen der Pulswellenform dienen kann, ist mit einer durchbrochenen Linie ebenfalls dargestellt. Bei einer Sympathikusdämpfung führt die Absenkung der kardialen Pumpleistung dazu, dass die Amplitude 53 des ersten Peaks des Pulswellensignals 61 im Vergleich zum Referenzsignal 41 erniedrigt ist. Die entsprechende Absenkung 62 des Peaks 51 kann von der Vorrichtung 10 auch quantitativ ermittelt werden. Darüber hinaus verlängert sich die späte Systole 57, da der zweite Peak 52 des Pulswellensignals 61 im Vergleich zum Referenzsignal 41 eine Verschiebung 63 zu einer relativ späteren Zeit aufweist, wenn die Pulswellensignale auf die gleiche zeitliche Dauer skaliert sind. Bei der über die Sympathikusdämpfung hervorgerufenen Blutdrucksenkung führt die Verringerung des peripheren Widerstands und der Nachlast somit zu einer Verschiebung 63 des zweiten Peaks in der Pulswellenform des Pulswellensignals 61.

Fig. 5 zeigt eine Pulswellenform 65 eines Pulswellensignals, das während einer Entspannungsübung erfasst wird, bei der eine Vagussteigerung erreicht wird. Die Pulswellenform des zu Beginn der Entspannungsübung erfassten Pulswellensignals 41, das als Referenz für die Analyse der Veränderungen der Pulswellenform dienen kann, ist mit einer durchbrochenen Linie ebenfalls dargestellt. Im Gegensatz zur Sympathikusdämpfung verändert sich die Pulswellenform in der späten Systole kaum, da bei der Vagussteigerung der periphere Widerstand und die Nachlast nicht so signifikant beeinflusst werden.

Sowohl bei einer Sympathikusdämpfung als auch bei einer Vagussteigerung verändert sich neben der Pulswellenform auch die Herzrate und somit die Dauer des Druck- oder Volumenpulses. Um dies zu berücksichtigen, sind die Pulswellenformen in Fig. 4 und Fig. 5 entlang der Zeitachse auf dieselbe Zeitdauer gestaucht dargestellt wie die als Referenz dienende Pulswellenform 41. Die Vorrichtung 10 kann die Veränderung der Herzrate ebenfalls rechnerisch berücksichtigen, indem die Dauer jedes Druck- oder Volumenpulses rechnerisch ermittelt und die Pulswellenform um einen Skalierungsfaktor entlang der Zeitachse gestreckt oder gestaucht wird, um eine Vergleichbarkeit mit einer Referenz-Pulswellenform 41 zu erleichtern.

Die Vorrichtung 10 kann eingerichtet sein, um zur Analyse der Pulswellenform eine Zeitableitung des Pulswellensignals zu ermitteln. Die Vorrichtung 10 kann eingerichtet sein, um sowohl eine erste Zeitableitung des Pulswellensignals als auch eine zweite Zeitableitung des Pulswellensignals zu ermitteln. Optional kann die Vorrichtung 10 eingerichtet sein, um zur Ermittlung der Dauer der frühen Systole und/oder der Dauer der späten Systole wenigstens eine erste Zeitableitung des EKG-Signals zu ermitteln. Anhand der Zeitableitungen lassen sich die Dauer der frühen Systole 56 und die Dauer der späten Systole 57 sowie optional eine Krümmung des Pulswellensignals in der späten Systole zuverlässig bestimmen.

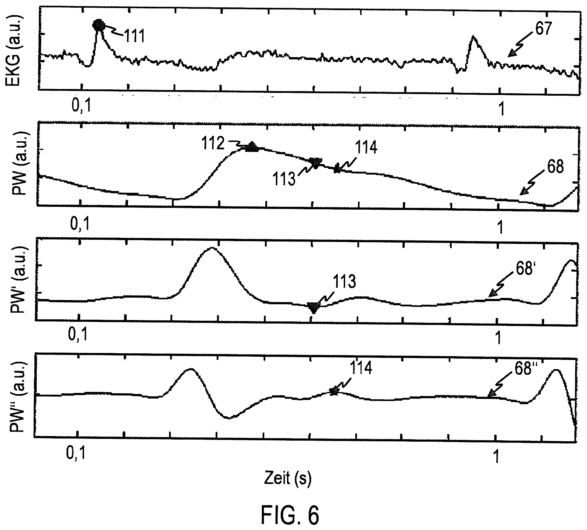

Fig. 6 zeigt ein EKG-Signal 67, ein Pulswellensignal 68, eine erste Zeitableitung 68' des Pulswellensignals 68 und eine zweite Zeitableitung 68" des Pulswellensignals 68 zur weiteren Erläuterung der Funktionsweise der Vorrichtung 10. Die Vorrichtung 10 kann eingerichtet sein, um einen Zeitpunkt einer R-Zacke des EKG-Signals 67 zu ermitteln. Dazu kann ein Maximum 111 des EKG-Signals identifiziert werden, wobei die Vorrichtung 10 hierzu optional eine erste Zeitableitung des EKG-Signals ermittelt. Ähnlich kann ein Maximum 112 des Pulswellensignals von der Vorrichtung 10 automatisch identifiziert werden.

Aus der ersten Zeitableitung 68' des Pulswellensignals und der zweiten Zeitableitung 68" des Pulswellensignals kann die Vorrichtung 10 das Ende der Systole 113 und die Krümmung 114 des Pulswellensignals in der späten systolischen Phase bestimmen.

Diese Zusatzinformation erlaubt potentielle Aussagen über Nachlastveränderungen und/oder Sympathikusaktivität. Eine die Nachlast quantifizierende Größe oder eine die Sympathikusaktivität quantifizierende Größe kann beispielsweise das Verhältnis der Dauer der späten Systole zur Dauer der frühen Systole, das Verhältnis der Dauer der späten Systole zur Gesamtdauer aus früher und später Systole und/oder das Verhältnis der Dauer der späten Systole zur Herzrate sein. Andere Verhältnisse oder andere aus der Dauer der frühen Systole, der Dauer der späten Systole und/oder der Herzrate abgeleitete Größen können ebenfalls herangezogen werden, um quantitative Information über eine Nachlastveränderungen und/oder Sympathikusaktivität zu gewinnen.

Die unter Bezugnahme auf Fig. 1 bis Fig. 6 beschriebenen Auswerteschritte zur Ermittlung der Pulswellenlaufzeit und Gewinnung zusätzlicher Information aus der Pulswellenform können für jeden Herzschlag einer Folge von Herzschlägen während einer Entspannungsübung ausgeführt werden. Dies kann die Erfassung und Auswertung der Pulswellenform sowie der Pulswellenlaufzeit über einen Zeitraum von wenigstens einer Minute, wenigstens fünf Minuten, wenigstens zehn Minuten oder wenigstens 15 Minuten beinhalten. Biofeedback, das von der Pulswellenlaufzeit sowie der Auswertung der Pulswellenform abhängt, kann während der Entspannungsübung ausgegeben und von Herzschlag zu Herzschlag aktualisiert werden.

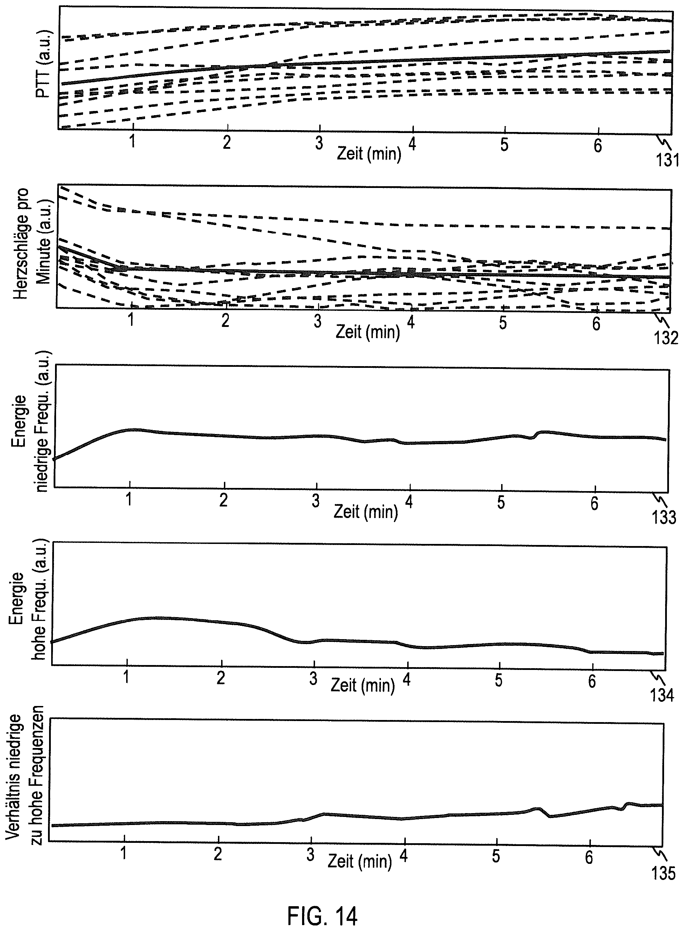

Zur Erzeugung von Biofeedback können die Pulswellenlaufzeit und optional die Herzrate weiterverarbeitet werden. Beispielsweise kann eine zeitliche Mittelung über einen vorgegebenen Zeitraum, der beispielsweise wenigstens eine Minute sein kann, vorgenommen werden, um die zeitlichen Mittelwerte der Pulswellenlaufzeit und optional der Herzrate als Biofeedback auszugeben. Alternativ oder zusätzlich kann die Energie des Pulswellensignals, des EKG-Signals oder daraus abgeleiteter Signale in verschiedenen Frequenzbereichen ermittelt werden. Dazu kann das Pulswellensignal und/oder das EKG-Signal einer Fouriertransformation unterzogen und aus dem in den Fourierraum transformierten Signal das spektrale Gewicht für wenigstens zwei voneinander verschiedene Frequenzbänder ermittelt werden, wie unter Bezugnahme auf Fig. 14 noch näher beschrieben wird. Die Vorrichtung 10 erlaubt es, über diese Auswertung der Pulswellenform und der Pulswellenlaufzeit, die indirekt Veränderungen des Blutdrucks anzeigt, individuell über visuelle und/oder akustische Signale die Blutdruckveränderung direkt und während einer Entspannungsübung an den Benutzer auszugeben. Dies bietet Verbesserungen im Vergleich zu den herkömmlichen Methoden des Biofeedbacks, die beispielsweise nur auf der Herzratenvariabilität oder herkömmlicher Pulswellenlaufzeitmessung beruhen.

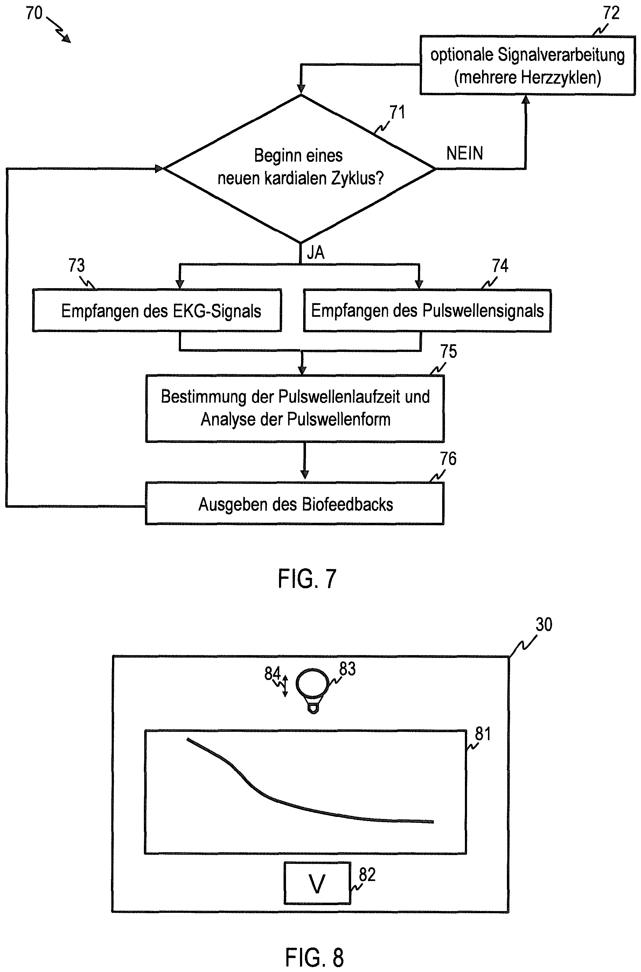

Fig. 7 ist ein Flussdiagramm eines Verfahrens 70, das von der Vorrichtung 10 nach einem Ausführungsbeispiel ausgeführt werden kann. Bei dem Verfahren kann für jeden Herzschlag einer Folge von Herzschlägen jeweils aus einem EKG-Signal und einem Pulswellensignal, das einen Druck- oder Volumenpuls in einem Blutkreislauf repräsentiert, die Pulswellenlaufzeit ermittelt und zusätzlich die Pulswellenform des Pulswellensignals ausgewertet werden. Bei dem Verfahren kann bei Schritt 71 überprüft werden, ob ein neuer kardialer Zyklus beginnt. Bei Schritten 73 und 74 können das EKG-Signal und das Pulswellensignal an wenigstens einer Schnittstelle von Sensoren 21, 22 empfangen werden. Bei Schritt 75 kann die Pulswellenlaufzeit bestimmt werden. Dazu kann der Zeitabstand zwischen einer R-Zacke des EKG-Signals und des Zeitpunkts, zu dem die steigende Flanke des Pulswellensignals einsetzt, ermittelt werden. Die Ermittlung der Pulswellenlaufzeit kann wie unter Bezugnahme auf Fig. 2 beschrieben vorgenommen werden. Bei Schritt 75 kann darüber hinaus die Pulswellenform analysiert werden. Dazu kann ermittelt werden, wie sich die Pulswellenform des Pulswellensignals, das während der Durchführung einer Entspannungsübung erfasst wird, im Vergleich zu der Pulswellenform wenigstens eines vorangegangen Pulswellensignals verändert. Zur Analyse der Pulswellenform des Pulswellensignals, das während der Durchführung einer Entspannungsübung erfasst wird, kann die Pulswellenform mit der Pulswellenform eines Referenz-Pulswellensignals verglichen werden, das beispielsweise zu Beginn der Entspannungsübung erfasst wird. Um auch bei veränderlicher Herzrate Änderungen der Pulswellenform zuverlässig und einfach erkennen zu können, kann eine Skalierung des Pulswellensignals entlang der Zeitachse vorgenommen werden, die die unterschiedlichen Dauern der zu unterschiedlichen Zeiten erfassten Pulswellensignale berücksichtigt. Die Analyse der Pulswellenform kann einen Vergleich der Signalstärke des Pulswellensignals, insbesondere an einem oder mehreren lokalen Maxima, beinhalten. Daraus lassen sich Rückschlüsse auf die kardiale Pumpleistung ziehen. Die Analyse der Pulswellenform kann einen Vergleich eines zweiten Peaks der Pulswellenform, der durch eine Reflexion des Druck- oder Volumenpulses in den peripheren Gefäßen verursacht wird, mit einem entsprechenden zweiten Peak in der Pulswellenform eines Referenz-Pulswellensignals beinhalten. Abhängig davon, ob sich die Pulswellenform in der späten Systole signifikant verändert, kann ermittelt werden ob eine Blutdruckveränderung über den Sympathikus oder den Vagus herbeigeführt wird. Bei Schritt 76 kann das Biofeedback ausgegeben werden. Das Biofeedback kann die Veränderung eines oder mehrerer Vitalparameter während einer Entspannungsübung als Funktion der Zeit und/oder als Funktion eines die Durchführung der Entspannungsübung charakterisierenden Parameters, beispielsweise einer Atemfrequenz, angeben. Das Biofeedback kann Information über eine Blutdruckveränderung, eine kardiale Pumpleistung, eine Pulswellenreflektion und/oder einen der Blutdruckveränderung zugrundeliegenden Wirkmechanismus beinhalten, ohne darauf beschränkt zu sein. Optional können auch Anweisungen für die Durchführung der Entspannungsübung ausgegeben werden. Die Anweisungen können abhängig von der Pulswellenlaufzeit und/oder der Analyse der Pulswellenform erzeugt werden. Das Biofeedback kann von Herzschlag zu Herzschlag aktualisiert werden. Dazu kann das Verfahren zu Schritt 71 zurückkehren. Parallel zu den Schritten 73-76 oder bis zum Beginn eines neuen kardialen Zyklus können bei Schritt 72 unterschiedliche zusätzliche Verarbeitungsschritte vorgenommen werden. Beispielsweise können gleitende Mittelwerte der Pulswellenlaufzeit berechnet und dem Benutzer als Biofeedback zur Verfügung gestellt werden.

Fig. 8 ist eine schematische Darstellung einer über eine optische Ausgabeeinheit 30 ausgegebenen Grafik, die von der Vorrichtung 10 erzeugt werden kann. Die Vorrichtung 10 kann die optische Ausgabeeinheit 30 ansteuern, um eine grafische Darstellung 81 einer Veränderung des Blutdrucks auszugeben. Die grafische Darstellung 81 kann die Veränderung der Pulswellenlaufzeit während der Entspannungsübung beinhalten. Die Veränderung des Blutdrucks kann über die Pulswellenlaufzeit als Funktion der Zeit oder als Funktion eines Parameters der Entspannungsübung dargestellt werden. Die Vorrichtung 10 kann die optische Ausgabeeinheit 30 ansteuern, um zusätzliche oder alternative Informationen 82 als Biofeedback auszugeben, die von der Analyse der Pulswellenform abhängen. Die zusätzliche Information kann Information über eine kardiale Pumpleistung, eine Pulswellenreflektion und/oder einen der Blutdruckveränderung zugrundeliegenden Wirkmechanismus beinhalten. Die Vorrichtung 10 kann die optische Ausgabeeinheit 30 optional ansteuern, um Anweisungen für die Durchführung der Atemübung auszugeben. Beispielsweise kann ein grafisches Symbol 83 ausgegeben werden, um den Benutzer zu veranlassen, im Rhythmus einer Bewegung 84 des Symbols 83 zu atmen. Alternativ oder zusätzlich kann die Vorrichtung 10 die Anweisungen auch über eine akustische Ausgabeschnittstelle, beispielsweise einen Lautsprecher, ausgeben.

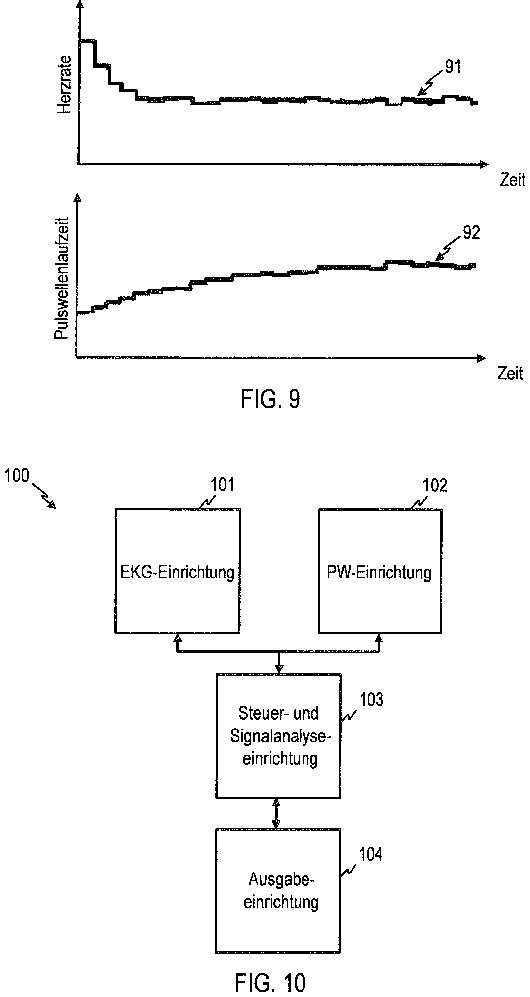

Fig. 9 zeigt die Veränderung einer Herzrate 91 und die Veränderung einer Pulswellenlaufzeit 92 während einer Entspannungsübung, gemittelt über mehrere Probanden. Wie Fig. 9 verdeutlicht, fällt die Herzrate 91 innerhalb eines relativ kurzen Zeitraums von wenigen Minuten nach Beginn der Entspannungsübung deutlich auf einen niedrigeren Wert ab und behält diesen dann bei. Die Änderung des Blutdrucks, die sich in der ermittelten Änderung der Pulswellenlaufzeit 92 zeigt, klingt jedoch erst deutlich nach dem Zeitpunkt ab, zu dem sich die Herzrate bereits auf einen erniedrigten Wert eingestellt hat. Fig. 9 verdeutlicht, dass die Herzrate und Pulswellenlaufzeit unterschiedliche Zeitabhängigkeiten aufweisen und daher eine Ermittlung der Pulswellenlaufzeit sowie eine Analyse der Pulswellenform für den Benutzer sinnvoll sind.

Fig. 10 ist ein schematisches Blockdiagramm eines Systems 100 nach einem Ausführungsbeispiel. Das System 100 umfasst einen EKG-Einrichtung 101 zur Erfassung des EKG-Signals, eine Einrichtung 102 zur Erfassung des Pulswellensignals, eine Steuer- und Signalanalyseeinrichtung 103 und eine Ausgabeschnittstelle 104. Die Steuer- und Signalanalyseeinrichtung 103 kann in ein Handgerät, beispielsweise einem Mobiltelefon, Tablet-Computer oder tragbaren Computer, integriert sein.

Die Steuer- und Signalanalyseeinrichtung 103 kann einerseits die Interaktion mit dem Bediener und andererseits die Koordination der Signal- und Datenflüsse innerhalb des Systems 100 kontrollieren. Die Steuer- und Signalanalyseeinrichtung 103 kann die Schnittstellen zu den Sensoren sowie die Prozessabläufe zur Signalverarbeitung definieren. In der Steuer- und Signalanalyseeinrichtung 103 werden die notwendigen Berechnungen vorgenommen, welche die Aussagen für zentrale Parameter auf Basis von peripher gemessenen Daten zulassen. Die Steuer- und Signalanalyseeinrichtung 103 kann zur Steuerung und Synchronisation aller internen Prozesse sowie der verschiedenen Datenschnittstellen eingerichtet sein.

Über eine optische und/oder akustische Ausgabeschnittstelle 104 kann die Steuer- und Signalanalyseeinrichtung 103 dem Benutzer eine individuell eingestellte Anweisung zur Durchführung der Entspannungsübung, beispielsweise eine Taktvorgabe für die Atmung, vorgeben. Bei einem Matra-Atmen kann diese Taktung beispielsweise mit zehn Atemzügen pro Minute beginnen und kann dann im Verlauf der nächsten Wochen langsam nach unten bis auf sechs Atemzüge pro Minute eingestellt werden. Über die optische und/oder akustische Ausgabeschnittstelle kann eine kontinuierliche Aufzeichnung der Pulswellenlaufzeit, die vom Blutdruck abhängt, sowie die Änderung der Pulswellenlaufzeit in Abhängigkeit von der Atemfrequenz ausgegeben werden. Ein Blutdruckabfall und damit ein Anstieg der Pulswellenlaufzeit kann über eine farbliche Kodierung oder mit einem entsprechenden akustischen Signal an den Benutzer ausgegeben werden.

Die Messung der Pulswellenlaufzeit kann kontinuierlich über eine (EKG) R-Zacken-getriggerte photoplethysmographische Messung, beispielsweise am Finger, erfolgen. Die System- und Messungssteuerung erfolgt vorteilhaft online, da die Steuerung wegen der transienten Natur des Herz-Kreislaufsystems zeitkritisch ist. Die Steuer- und/oder Regelvorgänge, die von der Steuer- und Signalanalyseeinrichtung 103 ausgeführt werden, können über das EKG-Signal getriggert werden, da es einerseits den immer wiederkehrenden Startpunkt des kardiovaskulären Kreislaufs markiert und andererseits robust über einen oder mehrere, beispielsweise bis zu sechs, Kanäle abgenommen werden kann.

Da die Signalverarbeitung zeit- und speicherkritisch ist, kann die Periodizität ausgenutzt und ein rekursives Informationsfenster zur Synchronisierung aller Signale definiert werden. Dieses Informationsfenster kann abhängig von der vorliegenden Eigenfrequenz des kardiovaskulären Systems, der Informationsausbreitung und der zurückzulegenden Distanzen im System sein. Die Eigenfrequenzen des menschlichen kardiovaskulären Systems liegen je nach Aktivierungszustand typischerweise zwischen 0,5 Hz und 3,5 Hz. Dies ist der Ausgangspunkt des transienten Verhaltens des Systems. Während der Entspannungsübung kann sich die Eigenfrequenz kaskadenartig ändern. Auch die Wellenausbreitungsgeschwindigkeit ändert sich im Zusammenhang mit der Aktivierung des Sympathikus oder Parasympathikus und bewegt sich typischerweise zwischen 4 m/s und 30 m/s. Die maximale Wellenlänge wird durch die Körpergröße der jeweiligen Person vorgegeben. Das Beobachtungsfenster, über das Veränderungen erfasst und rechnerisch weiter verarbeitet werden, hängt somit von mehreren Parametern ab, die benutzerdefiniert eingegeben oder von der erfindungsgemäßen Vorrichtung automatisch abgeschätzt werden können, um das Beobachtungsfenster so festzulegen, dass Auslöschungen vermieden und konsistente Informationsgewinnung sichergestellt werden kann. Eine Kaskadierung kann sich aus den unterschiedlichen Orten der Aufzeichnung und der unterschiedlichen Informationsausbreitung der Pulswelle ergeben, die von der Anatomie und dem Kreislaufzustand des Probanden abhängen kann.

Neben der Bestimmung der Pulswellenlaufzeit erlaubt die EKG-Einrichtung 101 auch die Erfassung der Herzfrequenz und/oder die Erfassung der Herzfrequenzvariabilität als Parameter der vegetativen Balance.

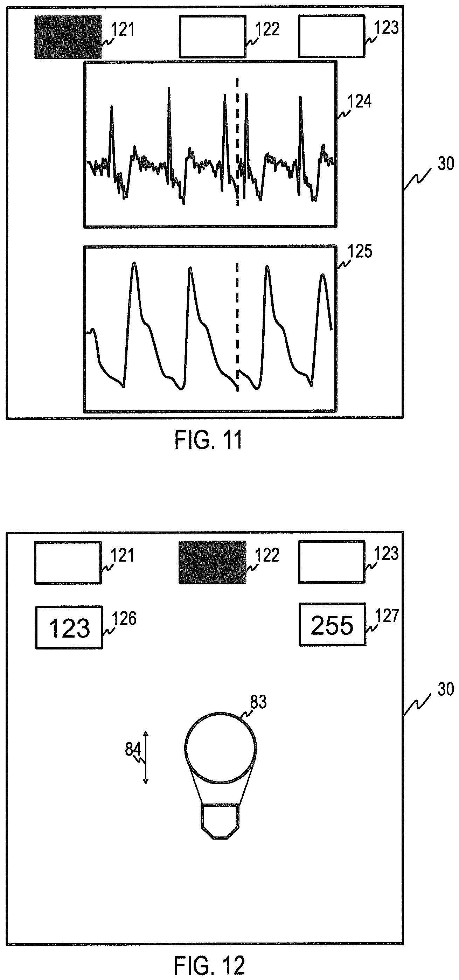

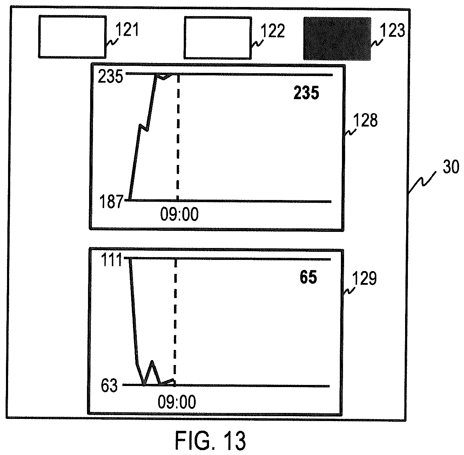

Fig. 11, Fig. 12 und Fig. 13 zeigen schematische Darstellung einer über eine optische Ausgabeeinheit 30 ausgegebenen Grafik, die von der Vorrichtung 10 erzeugt werden kann. Die Vorrichtung 10 kann die optische Ausgabeeinheit 30 ansteuern, um mehrere aktivierbare Bedienfelder 121-123 bereitzustellen.

Die Vorrichtung kann als Reaktion auf eine Benutzeraktivität, beispielsweise eine Auswahl eines Bedienfelds 121, ein Rohsignal 124 des ersten Sensors und/oder ein weiteres Rohsignal 125 des zweiten Sensors ausgeben. Das Rohsignal 124 kann das Ausgangssignal eines EKG-Sensors sein. Das weitere Rohsignal 125 kann das Ausgangssignal eines PPG-Moduls oder eines andere zur Laufzeitmessung geeigneten Sensors sein. Anhand der Ausgabe der Rohsignale 124, 125 kann der Benutzer einfach überprüfen, ob die Sensoren richtig am Körper angebracht und/oder die Verbindung zur Übertragung der Ausgangssignale hergestellt ist. Die Vorrichtung kann anhand der Rohsignale 124, 125 auch automatisch eine Plausibilitätsprüfung durchführen, um zu ermitteln, ob die Sensoren richtig angebracht und die Verbindung der Sensoren mit der Vorrichtung zur Signalübertragung korrekt hergestellt ist.

Die Vorrichtung kann als Reaktion auf eine Benutzeraktivität, beispielsweise eine Auswahl eines weiteren Bedienfelds 122, Anweisungen für die Durchführung der Atemübung auszugeben. Beispielsweise kann ein grafisches Symbol 83 ausgegeben werden, um den Benutzer zu veranlassen, im Rhythmus einer Bewegung 84 des Symbols 83 zu atmen. Alternativ oder zusätzlich kann die Vorrichtung 10 die Anweisungen auch über eine akustische Ausgabeschnittstelle, beispielsweise einen Lautsprecher, ausgeben. Gleichzeitig kann Biofeedback bereitgestellt werden. Beispielsweise können ein Basiswert 126 der Pulswellenlaufzeit, der der Pulswellenlaufzeit zu Beginn der Übung entspricht, und ein aktueller Wert 127 der Pulswellenlaufzeit angezeigt werden.

Die Vorrichtung kann als Reaktion auf eine Benutzeraktivität, beispielsweise eine Auswahl eines weiteren Bedienfelds 123, eine grafische Darstellung 128 einer Veränderung der Pulswellenlaufzeit ausgeben, die durch eine Veränderung des Blutdrucks bewirkt wird. Die grafische Darstellung 128 kann die Veränderung der Pulswellenlaufzeit während der Entspannungsübung und optional den aktuellen Wert der Pulswellenlaufzeit beinhalten. Die Veränderung des Blutdrucks kann über die Pulswellenlaufzeit als Funktion der Zeit oder als Funktion eines Parameters der Entspannungsübung dargestellt werden. Die optische Ausgabeeinheit 30 kann angesteuert werden, um zusätzliche Informationen 129 als Biofeedback auszugeben, die von der Analyse der Pulswellenform und/oder dem EKG-Signal abhängen. Die zusätzliche Information 129 kann beispielsweise die Herzrate als Funktion der Zeit während der Entspannungsübung darstellen.

Zusätzlich oder alternativ zur Erzeugung von Biofeedback während einer Entspannungsübung können die Vorrichtungen und Systeme nach Ausführungsbeispielen eingerichtet sein, Berichte zu erzeugen, die von in mehreren Entspannungsübungen erfassten und ausgewerteten Daten abhängen. Beispielsweise kann die Vorrichtung eingerichtet sein, um in jeder von mehreren Entspannungsübungen jeweils zu ermitteln, ob eine Nachlastveränderung erreicht wurde. Die Nachlastveränderung oder eine andere den Widerstand peripherer Gefäße quantifizierende Größe kann für jede der mehreren Entspannungsübungen nichtflüchtig gespeichert werden. Die entsprechenden Größen können durch eine Analyse der Pulswellenform ermittelt werden, wie dies oben beschrieben wurde. Bei Erzeugung eines Berichts nach einer größeren Zahl von Entspannungsübungen, die über einen Zeitraum von mehreren Tagen oder mehreren Wochen durchgeführt wurden, kann die Vorrichtung 10 Informationen erzeugen, die angeben, ob es dem Probanden gelungen ist, eine Senkung des Widerstands peripherer Gefäße zu erreichen.

Fig. 14 zeigt beispielhaft Informationen, die von der Vorrichtung 10 einzeln oder in Kombination als Biofeedback ausgegeben werden kann. Die Vorrichtung 10 kann einen zeitlichen Mittelwert der Pulswellenlaufzeit 131 ermitteln. Dabei kann die Vorrichtung 10 die von Herzschlag zu Herzschlag ermittelte Pulswellenlaufzeit über ein vorherbestimmtes Zeitintervall, das beispielsweise wenigstens eine Minute betragen kann, zeitlich mitteln. In Fig. 14 sind im Graphen für den zeitlichen Mittelwert der Pulswellenlaufzeit 131 die für individuelle Übungen jeweils ermittelten Mittelwerte der Pulswellenlaufzeit mit durchbrochenen Linien dargestellt. Der durch Mittelung über verschiedene Übungen bestimmte Mittelwert ist mit einer durchgezogenen Linie dargestellt.

Die Vorrichtung 10 kann optional auch einen zeitlichen Mittelwert der Herzrate 132 ermitteln. Dabei kann die Vorrichtung 10 die von Herzschlag zu Herzschlag ermittelte Herzrate über ein vorherbestimmtes Zeitintervall, das beispielsweise wenigstens eine Minute betragen kann, zeitlich mitteln. In Fig. 14 sind im Graphen für den zeitlichen Mittelwert der Herzrate 132 die für individuelle Übungen jeweils ermittelten Mittelwerte der Herzrate mit durchbrochenen Linien dargestellt. Der durch Mittelung über verschiedene Übungen bestimmte Mittelwert ist mit einer durchgezogenen Linie dargestellt.

Die Vorrichtung 10 kann auch komplexere Verarbeitungstechniken einsetzen. Beispielsweise kann die Vorrichtung 10 eingerichtet sein, die Energie des Pulswellensignals und/oder des EKG-Signals in einem, zwei oder mehr als zwei voneinander verschiedenen Frequenzbändern zu ermitteln. Dazu kann die Vorrichtung 10 beispielsweise das jeweilige Signal einer Fouriertransformation unterziehen. Zur Ermittlung der Energie in einem Frequenzband kann die Vorrichtung 10 das in den Frequenzraum fouriertransformierte Pulswellensignal und/oder EKG-Signal über das entsprechende Frequenzband numerisch integrieren. Andere Techniken zur Bestimmung der Energie des Pulswellensignals, des EKG-Signals oder daraus abgeleiteter Signale in einem, zwei oder mehr als zwei voneinander verschiedenen Frequenzbändern können eingesetzt werden.

Wie in Fig. 14 dargestellt, kann die Vorrichtung 10 die Energie 133 des Pulswellensignals und/oder EKG-Signals in einem ersten Frequenzband und die Energie 134 des Pulswellensignals und/oder EKG-Signals in einem zweiten Frequenzband ermitteln. Das erste Frequenzband kann ein Frequenzintervall (f₁₁, f₁₂) sein, und das zweite Frequenzband kann ein Frequenzintervall (f₂₁, f₂₂) sein, wobei f₁₁ < f₁₂ < f₂₁ < f₂₂. Die Vorrichtung 10 kann ermitteln, ob die Energie 133 im ersten Frequenzband, d.h. bei niedrigeren Frequenzen, während der Entspannungsübung zunimmt, und/oder ob die Energie 134 im zweiten Frequenzband, d.h. bei höheren Frequenzen, während der Entspannungsübung abnimmt. Entsprechend kann die Energie 133, 134 in wenigstens einem Frequenzband als Biofeedback verwendet und ausgegeben werden. Alternativ oder zusätzlich kann ein Verhältnis 135 der Energie 133 im ersten Frequenzband zur Energie 134 im zweiten Frequenzband von der Vorrichtung 10 bestimmt werden.

Die Vorrichtung 10 kann auch eingerichtet sein, um alternative oder zusätzliche Verarbeitungstechniken zur Erzeugung des Biofeedbacks abhängig von der Pulswellenlaufzeit und/oder der Pulswellenform von Pulswellen zu erzeugen.

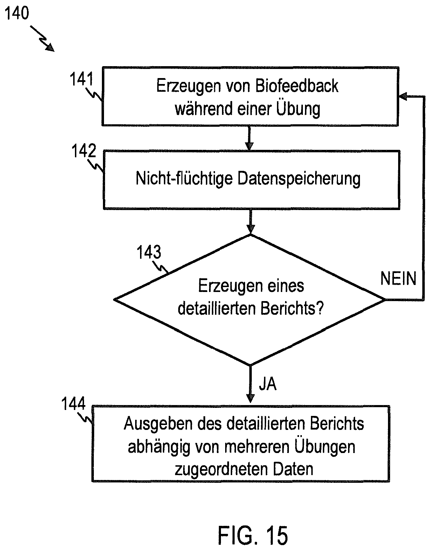

Fig. 15 ist ein Flussdiagramm 140 eines Verfahrens nach einem Ausführungsbeispiel. Bei Schritt 141 wird während einer Entspannungsübung Biofeedback erzeugt. Das während der Entspannungsübung erzeugt Biofeedback kann von der Pulswellenlaufzeit sowie optional von der Herzrate und/oder einer Analyse der Pulswellenform des Pulswellensignals abhängen. Bei Schritt 142 können Daten, die der Entspannungsübung zugeordnet sind, nichtflüchtig gespeichert werden. Die nichtflüchtig gespeicherten Daten können abhängig von der Analyse der Pulswellenform während der Entspannungsübung erzeugt werden. Die nichtflüchtig gespeicherten Daten können quantitative Informationen über eine Nachlastveränderung oder andere quantitative Informationen, die vom Widerstand peripherer Blutgefäße abhängen, aufweisen. Bei Schritt 143 kann überprüft werden, ob ein detaillierter Bericht zur Ausgabe an den Probanden und/oder eine den Probanden betreuende Fachperson erzeugt werden soll. Ein detaillierter Bericht kann beispielsweise erzeugt werden, wenn eine vordefinierte Anzahl von Entspannungsübungen unter Verwendung der Vorrichtung ausgeführt wurde. Alternativ oder zusätzlich kann ein detaillierter Bericht erzeugt werden, wenn über einen vordefinierten Zeitraum hinweg Entspannungsübungen unter Verwendung der Vorrichtung ausgeführt wurde. Alternativ oder zusätzlich kann ein detaillierter Bericht erzeugt werden, wenn der Proband oder eine den Probanden betreuende Fachperson diesen durch eine Benutzereingabe an der Vorrichtung anfordert. Falls ein detaillierter Bericht erzeugt werden soll, wird dieser bei Schritt 144 über eine Schnittstelle ausgegeben. Die Ausgabe des detaillierten Berichts kann die Ausgabe von abhängig von der Analyse der Pulswellenform erzeugten Informationen beinhalten, die angeben, ob es dem Probanden gelungen ist, eine Senkung des Widerstands peripherer Gefäße zu erreichen. Dazu kann beispielsweise die Veränderung der Nachlast über mehrere Entspannungsübungen ermittelt und ausgewertet werden.

Zur Verwendung der Systeme 1, 100 kann der Benutzer sowohl den EKG-Sensor, der beispielsweise als 1-Kanal-EKG-Sensor ausgestaltet sein kann, als auch das photoplethysmographische Modul beispielsweise am Finger befestigen. Das EKG-Signal und das Pulswellensignal werden erfasst. Aus dem Offset wird kontinuierlich die Pulswellenlaufzeit ermittelt.

Zu Beginn einer Atemübung kann der Benutzer beispielsweise mit einem normalen Atemrhythmus von 10-12 Atemzügen pro Minute atmen. Die Vorrichtung 10 kann die Entspannungsübung so führen, dass die Taktung langsam zu niedrigeren Atemfrequenzen verschoben wird, bis beispielsweise eine Frequenz von sechs Atemzügen pro Minute erreicht wird. Durch die Entspannungsübung wird der Blutdruck gesenkt, wobei Biofeedback an den Benutzer bereitgestellt wird. Die Atemfrequenz und das Biofeedback können über Audiosignale und/oder visuelle Signale übermittelt werden.

Die Vorrichtung kann beispielsweise bei häuslichen Entspannungsübungen verwendet werden, deren Dauer jeweils 10-20 Minuten täglich, in der Woche kumulativ mindestens 60 Minuten betragen kann. Eine Reduktion des Blutdruckes mit Effektgrößen von ungefähr 5 mmHg kann beispielsweise nach ein bis zwei Monaten erreicht werden.

Während Ausführungsbeispiele unter Bezugnahme auf die Figuren beschrieben wurden, können Abwandlungen bei weiteren Ausführungsbeispielen realisiert werden. Während die Vorrichtungen, Systeme und Verfahren Biofeedback ohne Verwendung einer Blutdruckmessmanschette bereitstellen können, können die Vorrichtungen, Systeme und Verfahren auch mit einer derartigen Blutdruckmessmanschette verwendet werden. Dabei kann beispielsweise unter Verwendung der Blutdruckmessmanschette vor Beginn einer Entspannungsübung oder nur zum Beginn einer Entspannungsübung eine Kalibrierung derart vorgenommen werden, dass die Pulswellenlaufzeit in Beziehung zu einem Absolutwert des Blutdrucks gesetzt werden kann. Dies ermöglicht die Ausgabe nicht nur relativer Blutdruckveränderungen während der Entspannungsübung, sondern optional auch von Absolutwerten des Blutdrucks.

Während Ausführungsbeispiele unter Bezugnahme auf die Erzeugung von Biofeedback bei geführten Atemübungen, beispielsweise Mantra-Atmen, beschrieben wurden, können die Vorrichtungen, Systeme und Verfahren zum Erzeugen von Biofeedback auch bei anderen Entspannungsübungen eingesetzt werden.

Vorrichtungen, Systeme und Verfahren nach Ausführungsbeispielen können das EKG-Signal nicht nur zur Bestimmung der Pulswellenlaufzeit verwenden. Vorrichtungen, Systeme und Verfahren nach Ausführungsbeispielen können das EKG-Signal sowohl zur Bestimmung der Pulswellenlaufzeit als auch zur Bestimmung der Herzratenvariabilität verwenden. Die Herzratenvariabilität kann als Biofeedback beispielsweise zusätzlich zur Veränderung der Pulswellenlaufzeit ausgegeben werden. Vorrichtungen, Systeme und Verfahren nach Ausführungsbeispielen können zur Bereitstellung von Biofeedback, insbesondere bei Entspannungsübungen zur Senkung des Blutdrucks bei milder Hypertonie, verwendet werden, ohne darauf beschränkt zu sein.

## Patentansprüche

1. Vorrichtung (10; 103, 104) zur Erzeugung von Biofeedback, insbesondere bei einer Entspannungsübung zur Blutdrucksenkung, aufweisend:
wenigstens eine Schnittstelle (12, 13) zum Empfangen eines Pulswellensignals (41; 61; 65), das einen Druck- oder Volumenpuls einer Pulswelle in einem Blutkreislauf als Funktion der Zeit repräsentiert, und eines EKG-Signals (42; 67),
eine Auswerteeinrichtung (11; 103), die eingerichtet ist, basierend auf dem Pulswellensignal (41; 61; 65; 68) und dem EKG-Signal (42; 67) eine Pulswellenlaufzeit (43) zu bestimmen, eine Auswertung einer Pulswellenform des Pulswellensignals (41; 61; 65; 68) auszuführen und das Biofeedback abhängig von der Pulswellenlaufzeit (43) und/oder der Auswertung der Pulswellenform zu erzeugen, wobei die Auswerteeinrichtung (11; 103) eingerichtet ist, durch die Auswertung der Pulswellenform zu ermitteln, ob eine durch eine Vagussteigerung oder eine Sympathikusdämpfung verursachte Blutdruckveränderung vorliegt, und
eine Ausgabeschnittstelle (14, 30; 104) zum Bereitstellen des Biofeedbacks (31; 81, 82; 126, 127, 128, 129).

2. Vorrichtung nach Anspruch 1, wobei die Auswerteeinrichtung (11; 103) eingerichtet ist, durch die Auswertung der Pulswellenform einen einer Blutdruckveränderung zugrundeliegenden Wirkmechanismus, eine Magnitude der Blutdruckveränderung und/oder eine Veränderung einer Pulswellenreflektion zu bestimmen.

3. Vorrichtung nach Anspruch 1 oder Anspruch 2, wobei die Auswerteeinrichtung (11; 103) eingerichtet ist, durch die Auswertung der Pulswellenform eine Nachlastveränderung zu ermitteln,
wobei optional die Auswerteeinrichtung (11; 103) eingerichtet ist, um über die Ausgabeschnittstelle (14, 30; 104) die Nachlastveränderung auszugeben, und/oder um über die Ausgabeschnittstelle (14, 30; 104) anzuzeigen, ob eine durch eine Vagussteigerung oder eine Sympathikusdämpfung verursachte Blutdruckveränderung vorliegt.

4. Vorrichtung nach einem der vorhergehenden Ansprüche, wobei die Auswerteeinrichtung (11; 103) eingerichtet ist, eine Veränderung wenigstens einer der folgenden Größen zu erkennen, um zu ermitteln, ob eine durch eine Vagussteigerung oder eine Sympathikusdämpfung verursachte Blutdruckveränderung vorliegt:
Dauer einer frühen systolischen Phase (56);
Druck in der frühen systolischen Phase (53); und/oder
Dauer einer späten systolischen Phase (57),
wobei die Vorrichtung optional eingerichtet ist, um eine erste Zeitableitung und eine zweite Zeitableitung (68'; 68") des Pulswellensignals (41; 61; 65) zu ermitteln.

5. Vorrichtung nach einem der vorhergehenden Ansprüche, wobei die Auswerteeinrichtung (11; 103) eingerichtet ist, durch die Auswertung der Pulswellenform eine Veränderung einer kardialen Pumpleistung zu bestimmen.

6. Vorrichtung nach einem der vorhergehenden Ansprüche, wobei die Auswerteeinrichtung (11; 103) eingerichtet ist, die Bestimmung der Pulswellenlaufzeit (43) und/oder die Auswertung der Pulswellenform jeweils für jeden Herzzyklus einer Mehrzahl aufeinanderfolgender Herzzyklen auszuführen,
wobei optional die Vorrichtung (10; 103, 104) eingerichtet ist, für jeden Herzzyklus der Mehrzahl aufeinanderfolgender Herzzyklen jeweils das Biofeedback (31; 81, 82; 126, 127, 128, 129) über die Ausgabeschnittstelle (14, 30; 104) auszugeben.

7. Vorrichtung nach einem der vorhergehenden Ansprüche, wobei das Biofeedback (31; 81, 82) wenigstens eine Information aufweist, die ausgewählt ist aus der Gruppe bestehend aus: einer Blutdruckveränderung, einem Blutdruck, einer kardialen Pumpleistung, einer Nachlastveränderung, einer Pulswellenreflektion und einem der Blutdruckveränderung zugrundeliegenden Wirkmechanismus.

8. Vorrichtung nach einem der vorhergehenden Ansprüche, wobei die Vorrichtung (10; 103, 104) eingerichtet ist, über die Ausgabeschnittstelle (14, 30; 104) sowohl das Biofeedback (31; 81, 82; 126, 127, 128, 129) als auch Anweisungen zur Durchführung der Entspannungsübung, insbesondere Atemanweisungen, auszugeben,
wobei optional die Vorrichtung (10; 103, 104) eingerichtet ist, die Anweisungen zur Durchführung der Entspannungsübung abhängig von der Pulswellenlaufzeit (43) und/oder der Auswertung der Pulswellenform zu erzeugen, und/oder
wobei optional die Ausgabeschnittstelle (14, 30; 104) eine optische Ausgabeeinheit (30) aufweist, wobei die Vorrichtung (10; 103, 104) eingerichtet ist, die Anweisungen zur Durchführung der Entspannungsübung (83) und das Biofeedback (31; 81, 82; 126, 127, 128, 129) gleichzeitig über die optische Ausgabeeinheit (30) auszugeben.

9. Vorrichtung nach einem der vorhergehenden Ansprüche, wobei die Auswerteeinrichtung (11; 103) eingerichtet ist, die Pulswellenlaufzeit (43) und/oder wenigstens eine durch die Auswertung der Pulswellenform ermittelte Kenngröße der Pulswellenform als Funktion eines Parameters der Entspannungsübung, insbesondere einer Atemfrequenz, zu bestimmen.

10. Vorrichtung nach einem der vorhergehenden Ansprüche, wobei die Ausgabeschnittstelle (14, 30; 104) konfiguriert ist, das Biofeedback (31; 81, 82; 126, 127, 128, 129) als grafische Darstellung bereitzustellen, die eine Veränderung der Pulswellenlaufzeit (43) während der Entspannungsübung beinhaltet.

11. System (1; 100), mit:
der Vorrichtung (10; 103, 104) zur Erzeugung von Biofeedback nach einem der vorhergehenden Ansprüche und
einer Sensoreinrichtung (21, 22; 101, 102), die mit der wenigstens einen Schnittstelle (12, 13) der Vorrichtung (10; 103, 104) koppelbar ist und zum Erfassen des Pulswellensignals (41; 61; 65; 68) und des EKG-Signals (42; 67) eingerichtet ist.

12. System nach Anspruch 11, wobei die Sensoreinrichtung (21, 22; 101, 102) einen ersten Sensor (21; 102) zum Erfassen des Pulswellensignals (41; 61; 65; 68) und einen zweiten Sensor (22; 101) zum Erfassen des EKG-Signals (42; 67) aufweist,
optional aufweisend eine Steuereinheit (11; 103) zum Steuern des ersten Sensors (21; 102) und des zweitens Sensors (22; 101) und zum Synchronisieren des von dem ersten Sensor (21; 102) erfassten Pulswellensignals (41; 61; 65; 68) und des von dem zweiten Sensor (22; 101) erfassten EKG-Signals (42; 67).

13. Verfahren zur Erzeugung von Biofeedback (31; 81, 82; 126, 127, 128, 129), insbesondere bei einer Entspannungsübung zur Blutdrucksenkung, aufweisend die Schritte:
Empfangen eines Pulswellensignals (41; 61; 65; 68), das einen Druck- oder Volumenpuls einer Pulswelle in einem Blutkreislauf als Funktion der Zeit repräsentiert, und eines EKG-Signals (42; 67) an wenigstens einer Schnittstelle (12, 13),
Bestimmen einer Pulswellenlaufzeit (43) durch Auswertung des Pulswellensignals (41; 61; 65; 68) und des EKG-Signals (42),
Auswerten einer Pulswellenform des Pulswellensignals (41; 61; 65; 68), wobei durch das Auswerten der Pulswellenform ermittelt wird, ob eine Blutdruckveränderung durch eine Vagussteigerung oder eine Sympathikusdämpfung verursacht wird,
Erzeugen des Biofeedbacks (31; 81, 82; 126, 127, 128, 129) abhängig von der Pulswellenlaufzeit (43) und/oder der Auswertung der Pulswellenform und
Ausgeben des Biofeedbacks (31; 81, 82; 126, 127, 128, 129).

14. Verfahren nach Anspruch 13, wobei das Biofeedback (31; 81, 82; 126, 127, 128, 129) während einer geführten Entspannungsübung zur Blutdrucksenkung, insbesondere während einer Atemübung, erzeugt wird, und/oder
wobei das ausgegebene Biofeedback (82) anzeigt, ob die Blutdruckveränderung durch eine Vagussteigerung oder eine Sympathikusdämpfung verursacht wird.

15. Verfahren nach Anspruch 13 oder Anspruch 14, wobei das EKG-Signal (42; 67) sowohl zur Bestimmung der Pulswellenlaufzeit (43) als auch zur Bestimmung einer Herzratenvariabilität verwendet wird, und/oder
wobei das Verfahren von der Vorrichtung (10; 103, 104) nach einem der Ansprüche 1 bis 10 oder von dem System (1; 100) nach Anspruch 11 oder Anspruch 12 ausgeführt wird.

## Claims

1. Apparatus (10; 103, 104) for generating biofeedback, in particular during a relaxation exercise for lowering blood pressure, comprising:
at least one interface (12, 13) for receiving a pulse wave signal (41; 61; 65), that represents a pressure pulse or volume pulse of a pulse wave in a blood circulation system as a function of time, and an ECG signal (42; 67),
an evaluation device (11; 103) that is configured to determine, on the basis of the pulse wave signal (41; 61; 65; 68) and the ECG signal (42; 67), a pulse transit time (43), perform an evaluation of a pulse wave form of the pulse wave signal (41; 61; 65; 68), and generate the biofeedback depending on the pulse transit time (43) and/or the evaluation of the pulse wave form, wherein the evaluation device (11; 103) is configured to determine, by evaluating the pulse wave form, whether there is a change in blood pressure caused by vagus enhancement or sympathetic attenuation, and
an output interface (14, 30; 104) for providing the biofeedback (31; 81, 82; 126, 127, 128, 129).

2. Apparatus according to claim 1, wherein the evaluation device (11; 103) is configured to determine a mode of action underlying a change in blood pressure, a magnitude of the change in blood pressure and/or a change in pulse wave reflection by evaluating the pulse wave form.

3. Apparatus according to claim 1 or claim 2, wherein the evaluation device (11; 103) is configured to determine a change in afterload by evaluating the pulse wave form, wherein the evaluation device (11; 103) is optionally configured to output the change in afterload via the output interface (14, 30; 104), and/or to indicate via the output interface (14, 30; 104) whether there is a change in blood pressure caused by vagus enhancement or sympathetic attenuation.

4. Apparatus according to any one of the preceding claims, wherein the evaluation device (11; 103) is configured to detect a change in at least one of the following variables in order to determine whether there is a change in blood pressure caused by vagus enhancement or sympathetic attenuation:
duration of an early systolic phase (56);
pressure in the early systolic phase (53); and/or
duration of a late systolic phase (57),
wherein the apparatus is optionally configured to determine a first time derivative and a second time derivative (68'; 68") of the pulse wave signal (41; 61; 65).

5. Apparatus according to any one of the preceding claims, wherein the evaluation device (11; 103) is configured to determine a change in cardiac pumping capacity by evaluating the pulse wave form.

6. Apparatus according to any one of the preceding claims, wherein the evaluation device (11; 103) is configured to perform the respective determination of the pulse transit time (43) and/or the respective evaluation of the pulse wave form for each cardiac cycle of a plurality of successive cardiac cycles, wherein the apparatus (10; 103, 104) is optionally configured to output the respective biofeedback (31; 81, 82; 126, 127, 128, 129) via the output interface (14, 30; 104) for each cardiac cycle of the plurality of successive cardiac cycles.

7. Apparatus according to any one of the preceding claims, wherein the biofeedback (31; 81, 82) comprises at least one piece of information selected from the group consisting of: a change in blood pressure, a blood pressure, a cardiac pumping capacity, a change in afterload, a pulse wave reflection, and a mode of action underlying the change in blood pressure.

8. Apparatus according to any one of the preceding claims, wherein the apparatus (10; 103, 104) is configured to output the biofeedback (31; 81, 82; 126, 127, 128, 129) and instructions for performing the relaxation exercise, in particular breathing instructions, via the output interface (14, 30; 104), wherein the apparatus (10; 103, 104) is optionally configured to generate the instructions for performing the relaxation exercise depending on the pulse transit time (43) and/or the evaluation of the pulse wave form, and/or optionally wherein the output interface (14, 30; 104) comprises an optical output unit (30), wherein the apparatus (10; 103, 104) is configured to output the instructions for performing the relaxation exercise (83) and the biofeedback (31; 81, 82; 126, 127, 128, 129) at the same time via the optical output unit (30).

9. Apparatus according to any one of the preceding claims, wherein the evaluation device (11; 103) is configured to determine the pulse transit time (43) and/or at least one core characteristic of the pulse wave form determined by the evaluation of the pulse wave form as a function of a parameter of the relaxation exercise, in particular a breathing rate.

10. Apparatus according to any one of the preceding claims, wherein the output interface (14, 30; 104) is configured to provide the biofeedback (31; 81, 82; 126, 127, 128, 129) as a graphic illustration which includes a change of the pulse transit time (43) during the relaxation exercise.

11. System (1; 100), comprising:
the apparatus (10; 103, 104) for generating biofeedback according to any one of the preceding claims and
a sensor device (21, 22; 101, 102) which is couplable to the at least one interface (12, 13) of the apparatus (10; 103, 104) and configured to detect the pulse wave signal (41; 61; 65; 68) and the ECG signal (42; 67).

12. System according to claim 11, wherein the sensor device (21, 22; 101, 102) comprises a first sensor (21; 102) for detecting the pulse wave signal (41; 61; 65; 68) and a second sensor (22; 101) for detecting the ECG signal (42; 67), optionally comprising a controller (11; 103) for controlling the first sensor (21; 102) and the second sensor (22; 101) and for synchronizing the pulse wave signal (41; 61; 65; 68) detected by the first sensor (21; 102) and the ECG signal (42; 67) detected by the second sensor (22; 101).

13. Method for generating biofeedback (31; 81, 82; 126, 127, 128, 129), in particular during a relaxation exercise for lowering blood pressure, comprising the steps of:
receiving a pulse wave signal (41; 61; 65; 68), that represents a pressure pulse or volume pulse of a pulse wave in a blood circulation system as a function of time, and an ECG signal (42; 67) at at least one interface (12, 13),
determining a pulse transit time (43) by evaluating the pulse wave signal (41; 61; 65; 68) and the ECG signal (42),
evaluating a pulse wave form of the pulse wave signal (41; 61; 65; 68), wherein it is determined by the evaluation of the pulse wave form whether a change in blood pressure is caused by vagus enhancement or sympathetic attenuation,
generating the biofeedback (31; 81, 82; 126, 127, 128, 129) depending on the pulse transit time (43) and/or the evaluation of the pulse wave form, and
outputting the biofeedback (31; 81, 82; 126, 127, 128, 129).

14. Method according to claim 13, wherein the biofeedback (31; 81, 82; 126, 127, 128, 129) is generated during a guided relaxation exercise for lowering blood pressure, in particular during a breathing exercise, and/or wherein the output biofeedback (82) indicates whether the change in blood pressure is caused by vagus enhancement or sympathetic attenuation.

15. Method according to claim 13 or claim 14, wherein the ECG signal (42; 67) is used to determine the pulse transit time (43) and to determine a heart rate variability, and/or wherein the method is carried out by the apparatus (10; 103, 104) according to any one of claims 1 to 10 or by the system (1; 100) according to claim 11 or claim 12.

## Revendications

1. Dispositif (10 ; 103, 104) pour la production de biofeedback, en particulier lors de l'exercice d'une détente pour baisser la pression artérielle, présentant :
au moins une interface (12, 13) pour la réception d'un signal d'onde pulsatile (41 ; 61 ; 65), qui représente une impulsion de pression ou de volume d'une onde pulsatile dans une circulation sanguine en fonction du temps, et d'un signal ECG (42 ; 67),
un système d'évaluation (11 ; 103), qui est conçu pour définir, sur la base du signal d'onde pulsatile (41 ; 61 ; 65 ; 68) et du signal ECG (42 ; 67), un temps de propagation d'onde pulsatile (43), pour effectuer une évaluation d'une forme d'onde pulsatile du signal d'onde pulsatile (41 ; 61 ; 65 ; 68) et pour produire le biofeedback en fonction du temps de propagation d'onde pulsatile (43) et/ou de l'évaluation de la forme d'onde pulsatile, dans lequel le système d'évaluation (11 ; 103) est conçu pour déterminer par l'évaluation de la forme d'onde pulsatile si une modification de pression artérielle provoquée par une augmentation vagale ou une atténuation sympathique est présente, et
une interface de sortie (14, 30 ; 104) pour la fourniture du biofeedback (31 ; 81, 82 ; 126, 127, 128, 129).

2. Dispositif selon la revendication 1, dans lequel le système d'évaluation (11 ; 103) est conçu, par l'évaluation de la forme d'onde pulsatile, pour définir un mécanisme d'action à la base d'une modification de pression artérielle, une amplitude de la modification de pression artérielle et/ou une modification d'une réflexion d'onde pulsatile.

3. Dispositif selon la revendication 1 ou revendication 2, dans lequel le système d'évaluation (11 ; 103) est conçu pour déterminer par l'évaluation de la forme d'onde pulsatile une modification de postcharge,
dans lequel éventuellement le système d'évaluation (11 ; 103) est conçu pour sortir par l'intermédiaire de l'interface de sortie (14, 30 ; 104) la modification de postcharge, et/ou pour afficher par l'intermédiaire de l'interface de sortie (14, 30 ; 104) si une modification de pression artérielle provoquée par une augmentation vagale ou une atténuation sympathique est présente.

4. Dispositif selon l'une quelconque des revendications précédentes, dans lequel le système d'évaluation (11 ; 103) est conçu pour identifier une modification au moins d'une des grandeurs suivantes, pour déterminer si une modification de pression artérielle provoquée par une augmentation vagale ou une atténuation sympathique est présente :
durée d'une phase systolique précoce (56) ;
pression dans la phase systolique précoce (53) ; et/ou
durée d'une phase systolique tardive (57),
dans lequel le dispositif est éventuellement conçu pour déterminer une première dérivée temporelle et une deuxième dérivée temporelle (68' ; 68") du signal d'onde pulsatile (41 ; 61 ; 65).

5. Dispositif selon l'une quelconque des revendications précédentes, dans lequel le système d'évaluation (11 ; 103) est conçu pour définir par l'évaluation de la forme d'onde pulsatile une modification d'une puissance de pompage cardiaque.

6. Dispositif selon l'une quelconque des revendications précédentes, dans lequel le système d'évaluation (11 ; 103) est conçu pour effectuer la définition du temps de propagation d'onde pulsatile (43) et/ou l'évaluation de la forme d'onde pulsatile respectivement pour chaque cycle cardiaque d'une pluralité de cycles cardiaques successifs,
dans lequel éventuellement le dispositif (10 ; 103, 104) est conçu pour sortir pour chaque cycle cardiaque de la pluralité de cycles cardiaques successifs respectivement le biofeedback (31 ; 81, 82 ; 126, 127, 128, 129) par l'intermédiaire de l'interface de sortie (14, 30 ; 104).

7. Dispositif selon l'une quelconque des revendications précédentes, dans lequel le biofeedback (31 ; 81, 82) présente au moins une information qui est sélectionnée dans le groupe constitué par : une modification de pression artérielle, une pression artérielle, une puissance de pompage cardiaque, une modification de postcharge, une réflexion d'onde pulsatile et un mécanisme d'action à la base de la modification de pression artérielle.

8. Dispositif selon l'une quelconque des revendications précédentes, dans lequel le dispositif (10 ; 103, 104) est conçu pour sortir, par l'intermédiaire de l'interface de sortie (14, 30 ; 104) aussi bien le biofeedback (31 ; 81, 82 ; 126, 127, 128, 129) que des consignes pour la mise en oeuvre de l'exercice d'une détente, en particulier des consignes respiratoires,
dans lequel éventuellement le dispositif (10 ; 103, 104) est conçu pour produire les consignes pour la mise en oeuvre de l'exercice d'une détente en fonction du temps de propagation d'onde pulsatile (43) et/ou de l'évaluation de la forme d'onde pulsatile, et/ou
dans lequel éventuellement l'interface de sortie (14, 30 ; 104) présente une unité de sortie optique (30), dans lequel le dispositif (10 ; 103, 104) est conçu pour sortir les consignes pour la mise en oeuvre de l'exercice d'une détente (83) et le biofeedback (31 ; 81, 82 ; 126, 127, 128, 129) simultanément par l'intermédiaire de l'unité de sortie optique (30).

9. Dispositif selon l'une quelconque des revendications précédentes, dans lequel le système d'évaluation (11 ; 103) est conçu pour définir le temps de propagation d'onde pulsatile (43) et/ou au moins une grandeur caractéristique de la forme d'onde pulsatile déterminée par l'évaluation de la forme d'onde pulsatile en fonction d'un paramètre de l'exercice d'une détente, en particulier d'une fréquence respiratoire.

10. Dispositif selon l'une quelconque des revendications précédentes, dans lequel l'interface de sortie (14, 30 ; 104) est configurée pour fournir le biofeedback (31 ; 81, 82 ; 126, 127, 128, 129) en tant que représentation graphique, qui comporte une modification du temps de propagation d'onde pulsatile (43) pendant l'exercice d'une détente.

11. Système (1 ; 100), avec :
le dispositif (10 ; 103, 104) pour la production de biofeedback selon l'une quelconque des revendications précédentes et
un système de capteur (21, 22 ; 101, 102), qui peut être couplé à la au moins une interface (12, 13) du dispositif (10 ; 103, 104) et est conçu pour détecter le signal d'onde pulsatile (41 ; 61 ; 65 ; 68) et du signal ECG (42 ; 67).

12. Système selon la revendication 11, dans lequel le système de capteur (21, 22 ; 101, 102) présente un premier capteur (21 ; 102) pour détecter le signal d'onde pulsatile (41 ; 61 ; 65 ; 68) et un deuxième capteur (22 ; 101) pour détecter le signal ECG (42 ; 67),
éventuellement présentant une unité de commande (11 ; 103) pour commander le premier capteur (21 ; 102) et le deuxième capteur (22 ; 101) et pour synchroniser le signal d'onde pulsatile (41 ; 61 ; 65 ; 68) détecté par le premier capteur (21 ; 102) et le signal ECG (42 ; 67) détecté par le deuxième capteur (22 ; 101).

13. Procédé pour la production de biofeedback (31 ; 81, 82 ; 126, 127, 128, 129), en particulier lors de l'exercice d'une détente pour la baisse de pression artérielle, présentant les étapes de :
réception d'un signal d'onde pulsatile (41 ; 61 ; 65 ; 68), qui représente une impulsion de pression ou de volume d'une onde pulsatile dans une circulation sanguine en fonction du temps, et d'un signal ECG (42 ; 67) sur au moins une interface (12, 13),
définition d'un temps de propagation d'onde pulsatile (43) par évaluation du signal d'onde pulsatile (41 ; 61 ; 65 ; 68) et du signal ECG (42),
évaluation d'une forme d'onde pulsatile du signal d'onde pulsatile (41 ; 61 ; 65 ; 68), dans lequel par l'évaluation de la forme d'onde pulsatile il est déterminé si une modification de pression artérielle est provoquée par une augmentation vagale ou une atténuation sympathique,
production du biofeedback (31 ; 81, 82 ; 126, 127, 128, 129) en fonction du temps de propagation d'onde pulsatile (43) et/ou de l'évaluation de la forme d'onde pulsatile et
sortie du biofeedback (31 ; 81, 82 ; 126, 127, 128, 129).

14. Procédé selon la revendication 13, dans lequel le biofeedback (31 ; 81, 82 ; 126, 127, 128, 129) est produit pendant l'exercice d'une détente guidé pour baisser la pression artérielle, en particulier pendant un exercice respiratoire, et/ou
dans lequel le biofeedback (82) sorti indique si la modification de pression artérielle est provoquée par une augmentation vagale ou une atténuation sympathique.

15. Procédé selon la revendication 13 ou revendication 14, dans lequel le signal ECG (42 ; 67) est utilisé aussi bien pour la définition du temps de propagation d'onde pulsatile (43) que pour la définition d'une variabilité de fréquence cardiaque, et/ou
dans lequel le procédé est exécuté par le dispositif (10 ; 103, 104) selon l'une quelconque des revendications 1 à 10 ou par le système (1 ; 100) selon la revendication 11 ou revendication 12.
